# EUROPÄISCHE PATENTSCHRIFT

(11) **EP 2 722 008 B1**
(45) Veröffentlichungstag und Bekanntmachung des Hinweises auf die Patenterteilung: **17.01.2018**
(21) Anmeldenummer: 12188674.1
(22) Anmeldetag: 16.10.2012
(51) Int. Cl.: A61B 17/00

(54) **Düse zur Zufuhr von biologischem Material, insbesondere Zellen, medizinische Vorrichtung mit einer derartigen Düse, Verwendung einer Düse, Verfahren zum Mischen von Fluiden und Gerät**
Nozzle for feeding of biological material, in particular cells, medical device having such a nozzle, use of a nozzle, method for mixing fluids and apparatus
Buse pour l'alimentation de matériau biologique, notamment des cellules, dispositif médical doté d'une telle buse, utilisation d'une buse, procédé de mélange de fluides et appareil

(43) Veröffentlichungstag der Anmeldung: 23.04.2014
(73) Patentinhaber: Erbe Elektromedizin GmbH, 72072 Tübingen (DE)
(72) Erfinder: Fischer, Klaus, 72202 Nagold (DE); Szyrach, Mara, 8052 Zürich (CH); Fech, Andreas, 72072 Tübingen (DE); Enderle, Markus D., 72070 Tübingen (DE)
(74) Vertreter: Bohnenberger, Johannes

(56) Entgegenhaltungen:
- US-A- 6 047 861
- US-A1- 2006 280 690
- US-A1- 2008 161 757
- US-A1- 2012 158 048

## Beschreibung

Die Erfindung betrifft eine Düse zur Zufuhr von biologischem Material, insbesondere Zellen, gemäß dem Oberbegriff des Patentanspruchs 1. Ferner betrifft die Erfindung eine medizinische Vorrichtung, insbesondere ein chirurgisches Instrument, mit einer derartigen Düse sowie die Verwendung einer Düse, ein Verfahren zum Mischen von Fluiden und ein Gerät. Eine Düse der eingangs genannten Art ist beispielsweise aus EP 2 163 204 A1 bekannt.

EP 2 163 204 A1 beschreibt eine Düse zum Mischen und Verabreichen eines biologischen Klebstoffs, insbesondere Fibrinklebers. Die Düse umfasst zwei parallel in einer Sonde angeordnete Zufuhrkanäle, die in eine gemeinsame Mischkammer am proximalen Ende der Düse münden.

Bei der bekannten Düse münden die beiden Zufuhrkanäle parallel zueinander in die Mischkammer. Das hat zur Folge, dass sich in der Mischkammer ein einheitliches Druckniveau einstellt, dass sich auf alle Einzelströme, die in die Mischkammer gelangen, auswirkt. Da unterschiedliche Substanzen in die Mischkammer geführt werden, die sich folglich auch in ihrer Viskosität unterscheiden, ergeben sich schwankende Mischungsverhältnisse. Das Druckniveau in der Mischkammer stellt sich außerdem auf den relativ höheren Wert der Einzeldrücke der beiden über die separaten Zufuhrkanäle zugeführten Substanzen ein. Somit entsteht in der Mischkammer ein relativ hoher Druck, der bei besonderen Anwendungen, beispielsweise bei der Zufuhr von Zellmaterial, unerwünscht ist. Konkret können durch den hohen Druck biologische Substanzen geschädigt werden.

Bei der Düse gemäß EP 2 163 204 A1 ist in der Mischkammer ferner ein Einsatz angeordnet, der eine verbesserte Vermischung der beiden in die Mischkammer geführten Substanzen bewirken soll. Der Einsatz weist Ablenkelemente auf, wodurch die in die Mischkammer einströmenden Fluide in eine Rotation innerhalb der Mischkammer versetzt werden. So entstehen in beiden Fluiden Scherspannungen, die sich schädigend auf biologisches Material, insbesondere Zellen, auswirken können.

Das Dokument US2006/280690 offenbart teilweise die Merkmale, die in Anspruch 1 beansprucht sind. Die Erfindung wird in den Ansprüchen 1 und 14 definiert und weitere Ausführungsformen finden sich in den abhängigen Ansprüchen.

Der Erfindung liegt die Aufgabe zugrunde, eine Düse zur Zufuhr von biologischem Material, insbesondere Zellen, anzugeben, die eine verbesserte und druckschonende Vermischung von Fluidströmen ermöglicht. Ferner besteht die Aufgabe darin, eine Düse anzugeben, die auf einfache Weise zwischen unterschiedlichen Fluidstrahlformen umschaltbar ist. Es ist eine weitere Aufgabe der Erfindung, eine medizinische Vorrichtung, die ein chirurgisches Instrument mit einer derartigen Düse aufweist, sowie die Verwendung einer Düse, ein Verfahren zum Mischen von Fluiden und ein chirurgisches Gerät zur Steuerung und Regelung des chirurgischen Instruments anzugeben. Das erfindungsgemäße chirurgische Instrument kann in einem Endoskop zum Einsatz kommen.

Erfindungsgemäß wird diese Aufgabe im Hinblick auf die Düse zur druckschonenden Vermischung durch den Gegenstand des Patentanspruchs 1, alternativ durch den Gegenstand des Anspruchs 13 und im Hinblick auf die medizinische Vorrichtung durch den Gegenstand des Patentanspruchs 17 gelöst. Die Aufgabe wird im Hinblick auf die Verwendung durch den Gegenstand des Anspruchs 15, im Hinblick auf das Verfahren durch den Gegenstand des Anspruchs 16 und im Hinblick auf das Gerät durch den Gegenstand des Anspruchs 18 gelöst.

So beruht die Erfindung auf dem Gedanken, eine Düse zur Zufuhr von biologischem Material, insbesondere Zellen, mit einer Mischkammer anzugeben, die durch eine proximale Endfläche und eine von der proximalen Endfläche beabstandete distale Endfläche begrenzt ist. Die Düse weist wenigstens eine Düsenöffnung auf, die in der distalen Endfläche ausgebildet ist. Ferner umfasst die Düse wenigstens zwei Zufuhrkanäle, die in die Mischkammer münden. Dabei ist ein erster Zufuhrkanal in der proximalen Endfläche angeordnet und mündet koaxial zur Düsenöffnung in die Mischkammer. Ein zweiter Zufuhrkanal weist eine Eintrittsöffnung auf, die an der distalen Endfläche seitlich, insbesondere tangential, in die Mischkammer mündet.

Die erfindungsgemäße Düse weist zwei Besonderheiten auf, die eine verbesserte und druckschonende Vermischung zweier Fluide ermöglichen. Einerseits ist vorgesehen, dass der erste Zufuhrkanal koaxial zur Düsenöffnung in die Mischkammer mündet. Damit wird erreicht, dass der Fluidstrom, der über den ersten Zufuhrkanal in die Mischkammer gelangt, geradlinig, d.h. umlenkungsfrei, durch die Mischkammer zur Düsenöffnung geführt ist. Durch die erfindungsgemäße Anordnung des ersten Zufuhrkanals wird die Entstehung von Scherkräften innerhalb dieses Zufuhrkanals nahezu vermieden. Es wird vermieden, dass auf das über den ersten Zufuhrkanal in die Mischkammer geführte Fluid Scherkräfte wirken. Durch die Reduzierung der Entstehung von Scherkräften wird die Gefahr der Beschädigung von, beispielsweise Zellen, die das im ersten Zufuhrkanal geführte Fluid enthält, reduziert vorzugsweise nahezu vollständig eliminiert. Andererseits ist im Unterschied zu bekannten Düsen vorgesehen, dass zusätzlich zu dem koaxial angeordneten ersten Zufuhrkanal wenigstens ein zweiter Zufuhrkanal seitlich in die Mischkammer mündet. Die erfindungsgemäße Anordnung und die erfindungsgemäße Ausgestaltung der Zufuhrkanäle verbessert die Vermischung und ermöglicht zusätzlich, die Druckverhältnisse in der Mischkammer zu beeinflussen, vorzugsweise zu reduzieren. Indem das über den zweiten Zufuhrkanal zugeführte Fluid seitlich, insbesondere tangential, in die Mischkammer strömt, entsteht in der Mischkammer eine im Wesentlichen rotierende Strömung des über den zweiten Zufuhrkanal zugeführten zweiten Fluids gegenüber dem ersten Fluid, das über den koaxial zur Düsenöffnung angeordneten ersten Zufuhrkanal in die Mischkammer strömt. Dies führt zu einer besonders schonenden Durchmischung der beiden Fluide, die im Übrigen bei deutlich geringeren Zufuhrdrücken in mindestens einem der beiden Zufuhrkanäle erreicht wird, als dies bei bisher bekannten Düsen gleicher Baugröße der Fall ist.

Es hat sich als vorteilhaft für die schonende Zufuhr von biologischem Material erwiesen, wenn die Vermischung der Fluide unmittelbar vor der Düsenöffnung, d.h. möglichst kurz vor dem Austritt aus der Düse, erfolgt. Die erfindungsgemäße Düse ermöglicht dies, indem die Eintrittsöffnung des zweiten Zufuhrkanals an der distalen Endfläche in die Mischkammer mündet. Die Vermischung von Zellmaterial, beispielsweise in Lösung befindliche Zellen, mit einem Trägerfluid erfolgt also möglichst spät, d.h. insbesondere unmittelbar vor der Düsenöffnung. Die Zellen sind daher den Strömungsturbulenzen und dem Druck bei der Vermischung vergleichsweise kurzzeitig ausgesetzt. Dies schont die Zellen, da insbesondere der zeitliche Einfluss von schädigenden Scherspannungen reduziert ist.

Ein weiterer Vorteil der erfindungsgemäßen Düse ergibt sich durch die Anordnung der Eintrittsöffnung des zweiten Zufuhrkanals. Konkret ist vorgesehen, dass die Eintrittsöffnung seitlich in die Mischkammer mündet. Das über die seitliche Eintrittsöffnung in die Mischkammer strömende Fluid trifft also unter einem Winkel auf das axial einströmende Fluid aus dem ersten Zufuhrkanal. So wird der Fluidstrom des zweiten Fluids, das im zweiten Zufuhrkanal geführt ist, vor der Zufuhr in die Mischkammer umgelenkt. Die erfindungsgemäße Ausbildung der Eintrittsöffnung ermöglicht die Reduzierung des statischen Drucks des zweiten Fluids. Aufgrund dieser erfindungsgemäße Ausbildung der Eintrittsöffnung ist es möglich, dass die beiden Fluide, das zweite und das erste Fluid mit voneinander verschiedenen Drücken der Mischkammer zugeführt werden. Dies ist beispielsweise bei der Zufuhrkanalanordnung gemäß EP 2 163 204 A1 nicht der Fall.

Bei einer bevorzugten Variante der erfindungsgemäßen Düse ist vorgesehen, dass die Mischkammer eine Länge L aufweist, die höchstens 500 µm, insbesondere höchstens 250 µm, vorzugsweise höchstens 150 µm, beträgt. Besonders bevorzugt ist es, wenn die Länge L der Mischkammer höchstens 100 µm, insbesondere höchstens 75 µm, vorzugsweise höchstens 50 µm, weiter vorzugsweise höchstens 30 µm, beträgt. Die Länge L der Mischkammer entspricht dabei dem Abstand zwischen der proximalen Endfläche und der distalen Endfläche. Die vergleichsweise geringe Länge der Mischkammer bewirkt eine möglichst späte Vermischung der in die Mischkammer geführten Fluide, weil diese eine maximal lange Strecke in den Zufuhrkanälen getrennt geführt und möglichst knapp, vorzugsweise unmittelbar, vor der Düsenöffnung in der Mischkammer vermischt werden. Damit wird eine schonende Zufuhr von Zellen erreicht. Zusätzlich wird aufgrund der geringen Länge L der Mischkammer und der daraus folgenden kurzen Vermischungszeit die Vernetzung der zugeführten Fluide beeinflusst, vorzugsweise weitestgehend vermieden. Der Vernetzungsprozess der zugeführten Fluide beginnt zusammen mit dem Mischprozess, also zu dem Zeitpunkt, bei dem die Fluide aufeinandertreffen. Durch die Vernetzung der Fluide entsteht ein Fluidgemisch, mit veränderten Eigenschaften, beispielsweise deutlich höherer Viskosität, was zu einer Druckveränderung, insbesondere zu einer Druckerhöhung führt. Durch Vernetzung und die damit verbundene Zustandsänderung des Materials können beispielsweise polymerähnliche, elastische Stoffe entstehen, wodurch die Gefahr einer Düsenverstopfung erheblich steigt. Idealerweise sollte die Vernetzung der zugeführten Fluide erst nach deren Austritt aus der Düse beginnen. Deshalb wird erfindungsgemäß die Länge der Mischkammer und der Austrittsöffnung der Düse so klein wie möglich gehalten.

Die Länge der Mischkammer ist vorzugsweise kleiner, insbesondere sehr viel kleiner als die Gesamtlänge der Düse. Die Düse erstreckt sich vom distalen Ende der Zufuhrleitungen bis zur Austrittsfläche der Düsenöffnung. Mit anderen Worten weist die Düse eine Gesamtlänge auf, die sich durch den Abstand zwischen dem distalen Ende der Zufuhrleitung und einer distalen Abschlussfläche erstreckt, in der die Austrittsseite der Düsenöffnung angeordnet ist. Den größten Teil der Gesamtlänge der Düse, nehmen die getrennt geführten Zufuhrkanäle ein. Diese erstrecken sich vom distalen Ende der Zufuhrleitungen bis in die Eintrittskammer, die den Übergang in die Mischkammer bildet. Die Zufuhrkanäle sind insbesondere über den größten Teil der Gesamtlänge der Düse kontinuierlich getrennt geführt. Es besteht vorzugsweise keine Verbindung zwischen den Zufuhrkanälen auf der Strecke zwischen den Zufuhrleitungen und der Mischkammer. Die Zufuhrkanäle sind ausschließlich beim Eintritt in die Mischkammer zusammengeführt bzw. fluidverbunden.

Die Mischkammer und die Düsenöffnung erstrecken sich vorzugsweise entlang eines relativ kleineren Abschnitts der Gesamtlänge der Düse. Konkret kann die kumulierte Länge aus der Mischkammerlänge und der Düsenöffnungslänge höchstens einem Drittel, insbesondere höchstens einem Fünftel, vorzugsweise höchstens einem Zehntel, der Gesamtlänge der Düse entsprechen. Die Düsenöffnung kann durch einen koaxial zur Längsachse der Düse angeordneten Zylinder gebildet sein. Die Düsenöffnung kann auch einen Abschnitt aufweisen, der durch die Form eines in Richtung der distalen Endfläche der Düse sich vergrößernden Kegels gebildet ist.

An dieser Stelle wird darauf hingewiesen, dass sich die medizinischen Richtungsangaben proximal und distal auf den Anwender der Düse bzw. der medizinischen Vorrichtung mit der Düse als Bezugspunkt beziehen. Vom Anwender weiter entfernt gelegene Bauteile sind distal angeordnet. Dem Anwender nähere Bauteile sind hingegen proximal angeordnet.

Die seitliche Eintrittsöffnung kann eine Tiefe T aufweisen, die der Länge L der Mischkammer entspricht. Mit anderen Worten kann sich die seitliche Eintrittsöffnung über die gesamte Länge der Mischkammer erstrecken. Damit wird die Gestaltung einer sehr kleinen Mischkammer möglich, was für die schonende Zufuhr von biologischem Material, insbesondere Zellen, von Vorteil ist. Ferner wird auf diese Weise ein kompakter und konstruktiv einfacher Aufbau der Düse erreicht.

Gemäß einer anderen Ausführungsform der erfindungsgemäßen Düse ist die distale Endfläche der Mischkammer senkrecht zu einer Längsachse des zweiten Zufuhrkanals angeordnet. Dadurch wird ein kompakter Aufbau der Düse möglich. Das zweite Fluid, das durch den zweiten Zufuhrkanal strömt, wird aufgrund der senkrecht zur Längsachse angeordneten distalen Endfläche der Mischkammer, mit einem Winkel von 90° umgelenkt, bevor es der Mischkammer zugeführt wird.

Ein kompakter Aufbau der Düse kann ferner dadurch erreicht werden, dass die Zufuhrkanäle zumindest abschnittsweise parallel zueinander angeordnet sind.

Die seitliche Eintrittsöffnung weist ein rechteckiges Querschnittsprofil auf. Dabei kann eine der vier Seitenflächen des Querschnittsprofils durch die distale Endfläche der Mischkammer gebildet sein. Das rechteckige Querschnittsprofil ermöglicht einen vereinfachten konstruktiven Aufbau und eine vereinfachte Herstellung der Düse. Die Querschnittsfläche der Eintrittsöffnung kann sehr klein ausgebildet sein. Dies führt zu einer Erhöhung der Strömungsgeschwindigkeit des Fluids im zweiten Zufuhrkanal, wodurch der Druck, insbesondere der statische Druck des im zweiten Zufuhrkanal geführten zweiten Fluids vor der Mischkammer reduziert wird.

Bei einer speziellen Ausführungsform kann der vorgenannte Effekt dadurch verstärkt werden, dass sich die seitliche Eintrittsöffnung in Richtung zur Mischkammer verjüngt. Konkret kann die seitliche Eintrittsöffnung zwei gekrümmte Seitenflächen aufweisen, die zur Mischkammer konvergieren. Die gekrümmten Seitenflächen können rechtwinklig zur distalen Endfläche der Mischkammer angeordnet sein. Durch die Verjüngung, die in einer speziellen Ausgestaltung als zwei konvergierende, gekrümmte Seitenflächen ausgestaltet ist, wird erreicht, dass das im zweiten Zufuhrkanal strömende Fluid unmittelbar vor dem Einströmen in die Mischkammer beschleunigt wird. Dadurch sinkt der statische Druck des zweiten Fluids. Das zweite Fluid strömt daher mit einem relativ geringen Druck in die Mischkammer ein, so dass vermieden wird, dass das axial durch die Mischkammer strömende erste Fluid einem Druck ausgesetzt wird, der biologisches Material, insbesondere Zellen, schädigen kann. Die Verengung, die sich durch die zueinander konvergierenden Seitenflächen ergibt, bildet eine Beschleunigungsstrecke für das zweite Fluid, entlang derer der statische Druck des zweiten Fluids sinkt. Somit kann die Düse vorteilhaft dazu genutzt werden, Zellen, d.h. beispielsweise in Suspensionen befindliche Zellen, schonend einem Trägerfluid beizumischen. Das Gemisch kann anschließend über die Düsenöffnung die Düse verlassen. Das die Zellen umfassende Fluid strömt vorzugsweise durch den ersten Zufuhrkanal, der koaxial zur Düsenöffnung angeordnet ist. Das Trägermedium, insbesondere Trägerfluid, gelangt seitlich über die Eintrittsöffnung des zweiten Zufuhrkanals in die Mischkammer.

Bei einer weiteren bevorzugten Ausführungsform der erfindungsgemäßen Düse ist vorgesehen, dass eine äußere Seitenfläche der seitlichen Eintrittsöffnung kontinuierlich in eine Innenumfangsfläche der Mischkammer übergeht. Auf diese Weise wird das durch die Eintrittsöffnung strömende Fluid unter Reduzierung bzw. Minderung von Turbulenzen in die Mischkammer geleitet. Die Ausgestaltung der Eintrittsöffnung bewirkt, dass das Fluid, welches durch die Eintrittsöffnung fließt in laminarer Form in die Mischkammer eintritt. Das stellt eine gleichmäßige Einströmung und folglich eine gleichmäßige, schonende, graduelle Vermischung des zweiten Fluids mit dem ersten Fluid sicher. Als kontinuierlicher Übergang wird im Rahmen der Anmeldung ein stetiger, d.h. im Wesentlichen barrierefreier, Übergang bezeichnet. Die äußere Seitenfläche geht also im Wesentlichen bündig in die Innenumfangsfläche der Mischkammer über, insbesondere ohne Stufen, Kanten oder dergleichen Hürden aufzuweisen.

In einer weiteren Ausgestaltung der Düse ist wenigstens ein dritter Zufuhrkanal mit einer weiteren seitlichen Eintrittsöffnung vorgesehen. Die weitere seitliche Eintrittsöffnung kann an der distalen Endfläche seitlich in die Mischkammer münden. Insgesamt kann die Düse also drei Zufuhrkanäle umfassen, wobei zwei Zufuhrkanäle über seitliche Eintrittsöffnungen in die Mischkammer münden. Ein weiterer Zufuhrkanal gelangt axial, insbesondere koaxial zur Düsenöffnung, in die Mischkammer. Die seitlich in die Mischkammer geführten Zufuhrkanäle, d.h. der zweite und dritte Zufuhrkanal, weisen vorzugsweise Eintrittsöffnungen auf, die an der distalen Endfläche der Mischkammer angeordnet sind bzw. unmittelbar an die distale Endfläche der Mischkammer angrenzen. Durch den dritten Zufuhrkanal bzw. den wenigstens einen weiteren Zufuhrkanal wird die Anwendungsbreite der Düse erhöht. So können über die Düse mehr als zwei unterschiedliche Fluide - gleichzeitig oder zeitlich versetzt - vermischt und zugeführt werden. Die Erfindung ist im Übrigen nicht auf zwei oder drei Zufuhrkanäle beschränkt, sondern bezieht sich auch auf eine Düse, die mehr als drei, insbesondere vier, fünf, sechs, sieben oder acht Zufuhrkanäle aufweist. Entscheidend ist es, dass wenigstens einer der Zufuhrkanäle koaxial zu einer Düsenöffnung in die Mischkammer mündet.

Grundsätzlich können die Zufuhrkanäle und/oder die Mischkammer und/oder die Düsenöffnung einteilig oder mehrteilig ausgebildet sein. Eine einteilige Ausbildung zeichnet sich durch eine gute Fluiddichtigkeit aus. Demgegenüber weist eine mehrteilige Ausbildung von Zufuhrkanälen und/oder Mischkammer und/oder Düsenöffnung eine verbesserte Herstellbarkeit auf.

Die mehrteilige Ausbildung von Zufuhrkanälen und/oder Mischkammer und/oder Düsenöffnung kann beispielsweise dadurch erreicht werden, dass, wie es eine weitere bevorzugte Ausgestaltung der Erfindung vorsieht, die Mischkammer in einer Mischkammerplatte ausgebildet ist, die zwischen einer Düsenplatte und einem Kanalträger angeordnet ist. Der Kanalträger umfasst die wenigstens zwei Zufuhrkanäle. Alternativ kann vorgesehen sein, dass die Mischkammer in der Düsenplatte ausgebildet ist. Grundsätzlich können die Zufuhrkanäle bei der erfindungsgemäßen Düse in einem Kanalträger und/oder die Düsenöffnung in einer Düsenplatte ausgebildet sein, wobei die Mischkammer insbesondere in der Düsenplatte oder einer Mischkammerplatte ausgebildet ist. Die Mischkammerplatte ist zwischen der Düsenplatte und dem Kanalträger angeordnet. In einer weiteren Ausführung wird die Mischkammer in dem Kanalträger (distale Endfläche) ausgebildet.

Vorzugsweise weist die Mischkammerplatte eine Plattenstärke auf, die der Länge der Mischkammer, insbesondere der Tiefe der Eintrittsöffnung, entspricht. Die Mischkammerplatte kann einteilig, insbesondere integral, mit der Düsenplatte ausgebildet sein. Mit anderen Worten kann die Düsenplatte zumindest abschnittsweise die Mischkammerplatte bilden bzw. die Mischkammer kann in der Düsenplatte ausgebildet sein.

In einer andern Ausführungsform kann die Mischkammer in dem Kanalträger ausgebildet sein. Sie ist dann Bestandteil des Kanalträgers. Bei der distalen Endfläche des Kanalträgers und der distale Endfläche der Mischkammer kann es sich dann um ein und dieselbe Endfläche handeln. Es ist auch möglich, dass die distale Endfläche des Kanalträgers eine Ringnut aufweist, die die Düsenplatte aufnehmen kann.

Bei der erfindungsgemäßen Düse kann außerdem ein Temperierkanal vorgesehen sein, der derart angepasst ist, dass ein durch wenigstens einen Zufuhrkanal strömendes Fluid temperierbar ist. Vorzugsweise umfasst der Temperierkanal eine Zuleitung und eine Rückleitung, die zu einem geschlossenen Temperierkreislauf verbindbar sind. Der Temperierkanal kann unmittelbar benachbart zur Mischkammer und/oder zu den Zufuhrkanälen angeordnet sein. Vorzugsweise verläuft der Temperierkanal parallel zu den Zufuhrkanälen. Durch die Temperierung, d.h. Erwärmung oder Kühlung, der Düse, insbesondere der durch die Zufuhrkanäle strömenden Fluide bzw. wenigstens eines der durch die Zufuhrkanäle strömenden Fluide, kann beispielsweise die Viskosität der Fluide beeinflusst werden. So kann über eine Temperaturerhöhung die Viskosität wenigstens eines der durch die Zufuhrkanäle geführten Fluide reduziert werden. Das hat zur Folge, dass auch die in der Mischkammer auftretenden Scherkräfte reduziert werden. Ferner kann die Beeinflussung der Temperatur der einzelnen Fluide in den Zufuhrkanälen zu einer verbesserten Vermischung der Fluide bzw. zu einer verbesserten Vernetzung der in den Fluiden enthaltenen Substanzen führen.

Die Düse kann außerdem eine Zerstäuberplatte aufweisen. Die Zerstäuberplatte kann distal mit der Düsenplatte verbunden sein und eine koaxial zur Düsenöffnung angeordnete Zerstäuberöffnung aufweisen. Ferner kann wenigstens ein Zerstäuberkanal in der Zerstäuberplatte ausgebildet sein und die Zerstäuberöffnung mit einem Gaszufuhrkanal verbinden. Vorzugsweise mündet der Zerstäuberkanal tangential in die Zerstäuberöffnung. So kann dem aus der Düse austretenden Fluidstrahl zusätzlich Gas zugeführt werden, das eine Zerstäubung, insbesondere eine Aerosolbildung, des austretenden Fluids ermöglicht.

Gemäß einem weiteren Aspekt beruht die Erfindung auf dem Gedanken, eine Düse für medizinische Zwecke mit einer Mischkammer anzugeben, die durch eine proximale Endfläche und eine von der proximalen Endfläche beabstandete distale Endfläche begrenzt ist. Die Düse weist wenigstens eine Düsenöffnung auf, die in der distalen Endfläche angeordnet ist. Die Mischkammer weist eine im Wesentlichen zylinderförmige Innenkontur mit wenigstens zwei seitlich, insbesondere tangential in die Mischkammer mündenden Eintrittsöffnungen auf. Die Eintrittsöffnungen sind jeweils mit einem Zufuhrkanal fluidverbunden. Die Eintrittsöffnungen weisen unterschiedliche oder gleiche Querschnittsflächen auf.

Die mittlere Eintrittsgeschwindigkeit des Fluids bei gegebenem Volumenstrom, welches durch die Eintrittsöffnung in die Mischkammer fließt wird von der Querschnittsfläche der Eintrittsöffnung bestimmt. Somit ist es möglich, unterschiedliche oder gleiche Fluide mit unterschiedlichen oder gleichen Volumenströmen mit gleicher, identischer mittlerer Eintrittsgeschwindigkeit in die Mischkammer einzuleiten. Dazu wird das Verhältnis der Querschnittsflächen der Eintrittsöffnungen der unterschiedlichen Zufuhrkanäle entsprechend dem Verhältnis der Volumenströme der Fluide, die durch die unterschiedlichen Zufuhrkanäle fließen, ausgelegt. Mit anderen Worten werden zwei oder mehrere Fluide in einem definierten Volumenverhältnis, d.h. in einem festgelegten Verhältnis der Volumenströme zusammengeführt. Dabei werden die Querschnittsflächen der Eintrittsöffnungen so ausgelegt, dass unabhängig von dem Mischungsverhältnis bzw. unabhängig von den Größen der Fluidströme die gleiche mittlere Eintrittsgeschwindigkeit aller separat zugeführten Fluide in die Mischkammer erreicht wird. Wird beispielsweise ein Mischungsverhältnis von 1 zu 4 vom Fluid des zweiten Zufuhrkanals zu dem Fluid im dritten Zufuhrkanal benötigt erfordert dies eine 4 mal größere Querschnittsfläche der Eintrittsöffnung des dritten Zufuhrkanals, bei gleicher mittlerer Eintrittsgeschwindigkeit der Fluide in die Mischkammer. Bei einem Mischungsverhältnis von 1:1 sind die Querschnittsflächen der Eintrittsöffnungen gleich groß.

Um eine möglichst schonende Vermischung, der durch die radial nach außen versetzten Zufuhrkanäle fließenden Fluide, mit dem Fluid, welches durch den koaxial angeordneten Zufuhrkanal fließt zu erreichen, ist es erforderlich, dass die von außen zugeführten Fluide mit einer identischen mittleren Fließgeschwindigkeit in die Mischkammer eintreten. Dies ermöglicht eine turbulenzarme, im Idealfall eine turbulenzfreie Vermischung der Fluide in der Mischkammer.

Fluide zeichnen sich durch ihre chemische und oder biologische Zusammensetzung und die Konzentrationen ihrer Bestandteile aus. Gleiche Fluide bedeutet, gleiche chemische und oder biologische Zusammensetzung und gleiche Konzentrationen der Bestandteile.

Diese Art der Düse ermöglicht die Einstellung unterschiedlicher Fluidstrahlformen, d.h. die Form des aus der Düsenöffnung austretenden Fluidstrahls. Dies wird bei der erfindungsgemäßen Düse besonders einfach dadurch erreicht, dass mittels einer Steuerung bzw. Regelung eines Gerätes, an dem ein chirurgisches Instrument mit einer erfindungsgemäßen Düse angeschlossen ist, die Fluidzufuhr in den verschiedenen Zufuhrkanälen gesteuert bzw. geregelt wird. Zur Erzeugung eines Kegelstrahls beim Austritt aus der Düsenöffnung wird ein Fluid ausschließlich durch einen radial nach außen versetzten Zufuhrkanal, beispielsweise den zweiten oder dritten Zufuhrkanal, zugeführt. Dieser Kegelstrahl beziehungsweise die Wirkung dieses Kegelstrahls auf zu behandelndes Gewebe kann durch die zusätzliche Zuführung eines Fluids durch den ersten Zufuhrkanal verstärkt werden. Die alleinige Zuführung eines Fluids durch den ersten Zufuhrkanal erzeugt einen zylindrischen Vollstrahl.

An dieser Stelle wird darauf hingewiesen, dass sich die Querschnittsdimensionen der Eintrittsöffnungen nicht nur auf rechteckige Querschnitte, sondern allgemein auf mehreckige oder runde oder eine Kombination von eckigen und runden Querschnittsformen der Eintrittsöffnungen bezieht.

Mit der vorliegenden Anmeldung werden die Düse zur Zufuhr von biologischem Material und die Düse für medizinische Zwecke sowohl separat, d.h. getrennt voneinander, als auch in Kombination offenbart. Durch die zuvor erläuterte Düse zur Zufuhr von biologischem Material kann zusätzlich die Form, Kegelform, zylindrischer Vollstrahl oder eine Mischform beider Strahlformen des aus der Düsenöffnung austretende Fluidstrahls erzeugt werden.

Die im Zusammenhang mit der Düse zur Zufuhr von biologischem Material genannten Vorteile und bevorzugten Weiterbildungen gelten ebenfalls für die Düse für medizinische Zwecke, die eine einfache Einstellung von unterschiedlichen Fluidstrahlformen ermöglicht.

Ferner besteht ein weiterer Aspekt der Erfindung darin, eine medizinische Vorrichtung, insbesondere ein chirurgisches Instrument, mit einer der zuvor genannten Düsen anzugeben.

Die Erfindung wird im Folgenden anhand von Ausführungsbeispielen unter Bezugnahme auf die beigefügten, schematischen Zeichnungen näher erläutert. Darin zeigen:
- Fig. 1: eine perspektivische Explosionsdarstellung einer erfindungsgemäßen Düse mit drei Zufuhrkanälen gemäß einem bevorzugten Ausführungsbeispiel;
- Fig. 2a: eine Draufsicht auf eine Düsenplatte mit integrierter Mischkammer für eine erfindungsgemäße Düse gemäß einem weiteren bevorzugten Ausführungsbeispiel;
- Fig. 2b: eine Querschnittsansicht der Düsenplatte gemäß Fig. 2a entlang der Linie A-A;
- Fig. 3: eine Längsschnittansicht durch eine erfindungsgemäße Düse mit der Düsenplatte gemäß Fig. 2a und 2b;
- Fig. 4a: eine Draufsicht auf eine Düsenplatte mit integrierter Mischkammer gemäß einem weiteren bevorzugten Ausführungsbeispiel, wobei Temperierkanäle vorgesehen sind;
- Fig. 4b: eine Querschnittsansicht der Düsenplatte gemäß Fig. 4a entlang der Linie B-B;
- Fig. 5a: eine Draufsicht auf eine Düsenplatte mit integrierter Mischkante nach einem weiteren bevorzugten Ausführungsbeispiel, bei dem drei Eintrittsöffnungen in die Mischkammer vorgesehen sind;
- Fig. 5b: eine Querschnittsansicht der Düsenplatte gemäß Fig. 5a entlang der Linie C-C;
- Fig. 6: eine perspektivische Ansicht einer erfindungsgemäßen Düse nach einem weiteren bevorzugten Ausführungsbeispiel, wobei eine Zerstäuberplatte vorgesehen ist;
- Fig. 7: eine Draufsicht auf die Zerstäuberplatte gemäß Fig. 6;
- Fig. 8a: eine Draufsicht auf eine einteilig, nahtlos ausgebildete Düse 1 gemäß einem weiteren bevorzugten Ausführungsbeispiel;
- Fig. 8b: eine Querschnittsansicht der Düse 1 gemäß Fig. 8a entlang der Linie D-D;
- Fig. 9a: eine perspektivische Ansicht einer erfindungsgemäßen Düse 1 nach einem weiteren bevorzugten Ausführungsbeispiel, wobei die Zufuhrkanäle rohrförmig ausgebildet sind und
- Fig. 9b: eine Querschnittsansicht der Düse 1 gemäß Fig 9a entlang der Linie E-E.

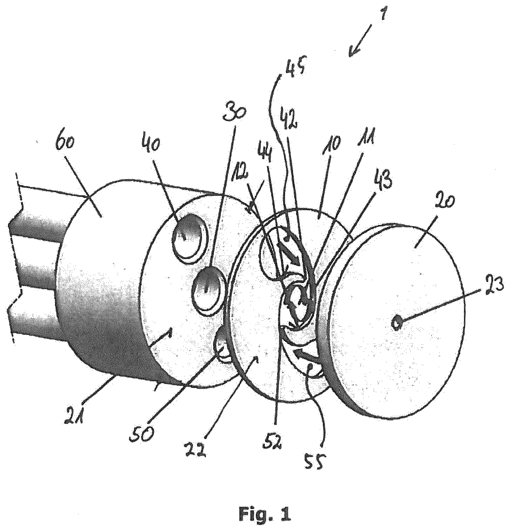

Die Düse 1 gemäß dem Ausführungsbeispiel nach Fig. 1 ist zur Zufuhr von biologischem Material geeignet und insbesondere zur Zufuhr von Zellen einsetzbar. Die Düse 1 kann Teil einer medizinischen Vorrichtung, insbesondere eines chirurgisches Instruments sein. Vorzugsweise ist die Düse 1 Teil eines Wasserstrahlapplikators, der insbesondere auch zur schonenden Zufuhr von Zellen geeignet ist.

Im Allgemeinen weist die Düse eine Mischkammer 11, eine Düsenöffnung 23 und wenigstens zwei Zufuhrkanäle 30, 40 auf. Die Zufuhrkanäle 30, 40 münden in die Mischkammer 11. Vorzugsweise münden die Zufuhrkanäle 30, 40 in einer proximalen Endfläche 21, die die Mischkammer 11 in proximaler Richtung begrenzt, in die Mischkammer 11. Die Mischkammer 11 weist ferner eine distale Endfläche 22 auf, die die Mischkammer 11 in distaler Richtung begrenzt. Mit anderen Worten ist die Mischkammer 11 zwischen der proximalen Endfläche 21 und der distalen Endfläche 22 ausgebildet. Ausgehend von der distalen Endfläche 22 beginnt die Düsenöffnung 23 und erstreckt sich von dieser fort.

In der proximalen Endfläche 21 mündet wenigstens ein erster Zufuhrkanal 30 in die Mischkammer 11. Der erste Zufuhrkanal 30 ist außerdem koaxial zur Düsenöffnung 23 angeordnet bzw. fluchtet mit der Düsenöffnung 23.

Ein zweiter Zufuhrkanal 40, der eine Eintrittskammer 45 umfasst, mündet, in Bezug auf den ersten Zufuhrkanal radial nach außen versetzt in die Mischkammer 11. Dazu ist vorgesehen, dass die Eintrittskammer 45 eine Eintrittsöffnung 42 aufweist, die, insbesondere tangential, in die Mischkammer 11 mündet. Insbesondere mündet der zweite Zufuhrkanal 40 über die Eintrittsöffnung 42 unmittelbar vor der Düsenöffnung 23 in die Mischkammer 11. Die Eintrittsöffnung 42 ist in Bezug auf die Düsenöffnung 23 radial nach außen, in Bezug auf die Eintrittskammer 45 radial nach innen angeordnet. Die Eintrittsöffnung 42 verbindet die Eintrittskammer 45 des Zufuhrkanals 40 mit der Mischkammer 11. Dies ist erfindungsgemäß derart realisiert, dass die Eintrittsöffnung 42 an der distalen Endfläche 22 in die Mischkammer 11 übergeht.

Konkret kann vorgesehen sein, dass der zweite Zufuhrkanal 40 eine Umlenkfläche 41 aufweist, die am distalen Ende der Eintrittskammer 45 des zweiten Zufuhrkanals 40 angeordnet ist. Die Umlenkfläche 41 begrenzt den zweiten Zufuhrkanal 40 in axialer Richtung und bewirkt insbesondere eine Umlenkung des im zweiten Zufuhrkanal 40 geführten Fluids. Die Umlenkfläche 41 geht ebenengleich in die distale Endfläche 22 der Mischkammer 11 über bzw. ist flächengleich mit der distalen Endfläche 22 ausgebildet. Grundsätzlich befinden sich die Umlenkfläche 41 und die distale Endfläche 22 in derselben Ebene. Die Umlenkfläche 41 und die distale Endfläche 22 bilden vorzugsweise einzelne, aneinander angrenzende Bereiche einer gemeinsamen Bauteiloberfläche.

Die Düse 1 gemäß Fig. 1 weist einen mehrteiligen Aufbau auf. Grundsätzlich kann die Düse auch einteilig aufgebaut sein. Insbesondere können die Zufuhrkanäle 30, 40, 50, die Mischkammer 11 und die Düsenöffnung 23 einteilig, nahtlos ausgebildet bzw. in einem einteiligen Bauteil integriert sein.

Die Düse gemäß Fig. 1 weist drei Zufuhrkanäle 30, 40, 50 auf, die in einem Kanalträger 60 angeordnet sind. Die Zufuhrkanäle 30, 40, 50 verlaufen vorzugsweise parallel zueinander im Kanalträger 60. Insbesondere sind die Zufuhrkanäle 30, 40, 50 in einer Reihe angeordnet, d.h. die Längsachsen der Zufuhrkanäle 30, 40, 50 schneiden eine gemeinsame Gerade, die quer zu den Längsachsen der Zufuhrkanäle 30, 40, 50 angeordnet ist. Der Kanalträger 60 weist an seinem distalen Ende eine Fläche auf, die die proximale Endfläche 21 bildet. Die proximale Endfläche 21 begrenzt die Mischkammer 11 in proximaler Richtung.

Die Mischkammer 11 ist bei dem Ausführungsbeispiel gemäß Fig. 1 in einer Mischkammerplatte 10 ausgebildet. Die Mischkammerplatte 10 liegt im montierten Zustand direkt auf der die Mischkammer 11 proximal begrenzenden proximalen Endfläche 21 auf, die dem Kanalträger 60 zugeordnet ist. Die Mischkammer 11 weist im Wesentlichen ein zylinderförmiges Profil auf. Konkret weist die Mischkammer 11 eine Innenumfangsfläche 12 auf, die abschnittsweise kreisbogenförmig gekrümmt ist. Die Innenumfangsfläche 12 ist durch die Eintrittsöffnungen 42, 52 unterbrochen.

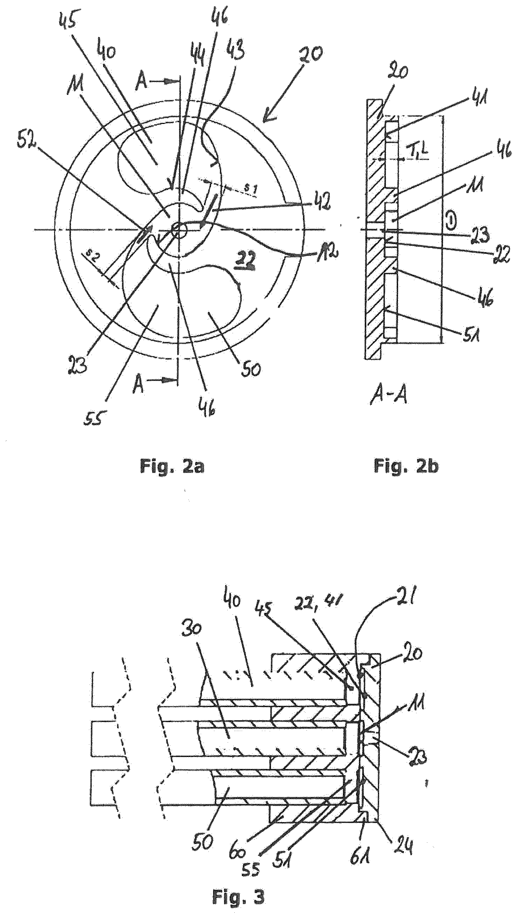

Die Innenumfangsfläche 12 der Mischkammer 11 geht kontinuierlich in eine äußere Seitenfläche 43 der Eintrittsöffnung 42 über. Die äußere Seitenfläche 43 kann quer, senkrecht, zu den Zufuhrkanälen 30, 40, 50 angeordnet sein. Die Anordnung und der kontinuierliche Übergang der Innenumfangsfläche 12 in die äußere Seitenfläche 43 gilt vorzugsweise für alle Eintrittsöffnungen 42, 52, die in die Mischkammer 11 münden. Die Eintrittsöffnungen 42, 52 sind, wie in den Fig. 2a, 4a und 5a gut erkennbar ist, im Wesentlichen als tropfenförmige Ausschnitte oder Durchbrüche in der Mischkammerplatte 10 oder der Düsenplatte 20 oder dem Kanalträger 60 angeordnet. Als äußere Seitenfläche 43 wird diejenige Seitenfläche des tropfenförmigen Ausschnitts bezeichnet, die kontinuierlich in die Innenumfangsfläche 12 übergeht, d.h. der Krümmungsradius der äußeren Seitenfläche 43 verändert sich kontinuierlich bis auf den Wert des Krümmungsradius der Innenumfangsfläche 12. Mit anderen Worten, der Krümmungsradius verändert sich beim Übergang von der äußeren Seitenfläche 43 der Eintrittsöffnung 42, 52 bis in die Innenumfangsfläche 12, d.h. einschließlich der Innenumfangsfläche 12, der Mischkammer 11 kontinuierlich. Der Krümmungsradius kann sich kontinuierlich verkleinern oder kontinuierlich vergrößern, er kann auch konstante Bereiche aufweisen. Der Übergang der Innenumfangsfläche 12 in die äußere Seitenfläche 43 ist stufenlos ausgebildet. Die Eintrittsöffnung 42, 52 weist ferner eine innere Seitenfläche 44 auf, die ebenfalls eine Krümmung aufweist und in der Innenumfangsfläche 12 endet. Die Krümmung der inneren Seitenfläche 44 weist einen Krümmungsradius auf, der sich von dem Krümmungsradius der äußeren Seitenfläche 43 unterschiedet. Vorzugsweise ist der Krümmungsradius der inneren Seitenfläche 44 kleiner als der Krümmungsradius der Seitenfläche 43 und verringert sich in Richtung der Innenumfangsfläche 12. Ausgehend von der Eintrittskammer 45 erstreckt sich die innere Seitenfläche 44 in Richtung der Eintrittsöffnung 42, und geht absatz- bzw. stufenlos in die Innenumfangsfläche 12 über, die in der äußeren Seitenfläche 43 der Eintrittskammer 55 mündet. Somit besteht zwischen einer Eintrittskammer 45, 55 und der Mischkammer 11 ein im Wesentlichen sichelförmig gewölbter Steg 46, zwischen dessen Spitze und der äußeren Seitenfläche 43 die Eintrittsöffnung 42, 52 angeordnet ist. Wie in Fig. 2a weiter gut erkennbar ist, konvergieren die äußere Seitenfläche 43 und die innere Seitenfläche 44 zueinander. Mit anderen Worten verengt sich die Eintrittsöffnung 42 bzw. die Eintrittskammer 45 in Richtung zur Eintrittsöffnung 42. Dasselbe gilt für die Eintrittskammer 55 und die Eintrittsöffnung 52. Die äußere Seitenfläche 43 und die innere Seitenfläche 44 bilden in radialer Richtung die seitlichen Begrenzungen der Eintrittskammern 45, 55. In axialer Richtung werden die Eintrittskammern 45, 55 durch die proximale Endfläche 21 und die distale Endfläche 22 begrenzt.

Grundsätzlich können die Eintrittsöffnungen 42, 52 unterschiedliche Breiten s1, s2 aufweisen. Durch unterschiedliche Breiten s1, s2 der Eintrittsöffnungen 42, 52, bei gleicher Tiefe T der Eintrittsöffnung können gleiche mittlere Strömungsgeschwindigkeiten bzw Fließgeschwindigkeiten beim Eintritt der Fluide in die Mischkammer 11 erzeugt werden. Um dies zu erreichen, ist wie oben beschrieben, die Kenntnis der vorgegebenen /geforderten Volumenstromverhältnisse der Fluide zueinander, die durch separate Eintrittsöffnungen 42,52, in die Mischkammer 11 strömen, erforderlich. Beispielweise kann vorgesehen sein, dass die erste Eintrittsöffnung 42 eine Breite s1 aufweist, die größer als die Breite s2 der zweiten Eintrittsöffnung 52 ist. Das hat zur Folge, dass das Fluid, das über die erste Eintrittsöffnung 42 mit einem Volumenstrom Q₁ in die Mischkammer 11 strömt, mit einer mittleren Fließgeschwindigkeit in die Mischkammer 11 fließt, welche mit der mittleren Fließgeschwindigkeit des zweiten Fluids, das über die zweite Eintrittsöffnung 52 mit der relativ schmaleren Öffnungsbreite mit einem Q₂ < Q₁ einströmt, identisch ist. Das fördert die schonende Vermischung der über den zweiten Zufuhrkanal 40 und den dritten Zufuhrkanal 50 zugeführten Fluide. Voraussetzungen für die identische mittlere Fließgeschwindigkeit beim Eintritt in die Mischkammer der beiden über die Eintrittsöffnungen 42, 52 zugeführten Fluide sind nicht nur die Querschnittsflächen der Eintrittsöffnungen sondern auch wie oben beschrieben die Verhältnisse bezüglich Volumenstrom.

Die Mischkammer 11 ist in distaler Richtung durch eine distale Endfläche 22 begrenzt. Die distale Endfläche 22 ist vorzugsweise in einer Düsenplatte 20 ausgebildet, die bei dem Ausführungsbeispiel gemäß Fig. 1 als separates Bauteil ausgebildet ist. Es ist auch möglich, wie die Fig. 2a und 2b beispielsweise zeigen, dass die Düsenplatte Aussparungen bzw. Vertiefungen in Form der Mischkammer 11 und der Eintrittskammern 45, 55 sowie Eintrittsöffnungen 42, 52 aufweist. Mit anderen Worten kann die Mischkammerplatte 10 integral mit der Düsenplatte 20 bzw. die Mischkammer 11 in der Düsenplatte 20 ausgebildet sein.

Die auf der proximalen Seite der Düsenplatte 20 angeordnete Fläche bildet die distale Endfläche 22 der Mischkammer 11. Gleichzeitig bildet die distale Endfläche 22, eine Umlenkfläche 41, 51 für die Zufuhrkanäle 40, 50. Das bedeutet, dass sowohl die Eintrittskammern 45, 55, als auch die Mischkammer 11 durch die distale Endfläche 22 bzw. die Umlenkfläche 41, 51 limitiert sind.

In der Düsenplatte 20 ist eine Düsenöffnung 23 ausgebildet. Die Düsenöffnung 23 ist koaxial zum ersten Zufuhrkanal 30 angeordnet. Insbesondere ist die Düsenöffnung 23 koaxial zur Mischkammer 11 und zum ersten Zufuhrkanal 30 angeordnet. Die Düsenöffnung 23 weist einen Querschnittsdurchmesser auf, der kleiner als der Querschnittsdurchmesser des ersten Zufuhrkanals 30 ist. Im Allgemeinen kann vorgesehen sein, dass der erste Zufuhrkanal 30 und die Mischkammer 11 im Wesentlichen denselben Querschnittsdurchmesser aufweisen. Tendenziell kann der Querschnittsdurchmesser der Mischkammer 11 einen etwas größeren Wert als der Querschnittsdurchmesser des ersten Zufuhrkanals 30 aufweisen. Der Querschnittsdurchmesser der Düsenöffnung 23 kann kleiner als der Querschnittsdurchmesser der Mischkammer 11 und der Querschnittsdurchmesser des Zufuhrkanals 30 sein.

Wie in Fig. 3 ferner erkennbar ist, sind sowohl die proximale Endfläche 21, als auch die distale Endfläche 22 bzw. die Umlenkflächen 41, 51 im Wesentlichen senkrecht zu den Längsachsen der Zufuhrkanäle 30, 40, 50 angeordnet. Im Allgemeinen können die Umlenkflächen 41, 51 sowie die distale Endfläche 22 und ggf. die proximale Endfläche 21 senkrecht zu einer Längsachse des zweiten Zufuhrkanals 40 angeordnet sein.

Es sind auch Ausführungsformen möglich, in der die distale Endfläche 22 in einem Winkel, der sich von 90 Grad unterscheidet, zur Längsachse der Düse 1 bzw. eines Zufuhrkanals 30, 40, 50 angeordnet ist. Die Endfläche 22 kann in einem Bereich von 10 bis 90 Grad vorzugsweise 25 bis 60 Grad angeordnet sein.

Ferner zeigt Fig. 1 anschaulich, dass die Eintrittsöffnungen 42, 52 jeweils eine Tiefe T aufweisen, die der Länge L der Mischkammer 11 entspricht. Bei der mehrteiligen Ausführungsform gemäß Fig. 1 entspricht die Tiefe T der Eintrittsöffnung 42, 52 und die Länge L der Mischkammer 11 der Plattenstärke der Mischkammerplatte 10. Grundsätzlich wird die Länge der Mischkammer 11 durch den Abstand der proximalen Endfläche 21 von der distalen Endfläche 22 bestimmt. Ebenso wird die Tiefe T der Eintrittsöffnung 42, 52 bestimmt, d.h. der Abstand zwischen der proximalen Endfläche 21 und der distalen Endfläche 22 entspricht der Tiefe T der Eintrittsöffnung 42, 52.

Die Eintrittskammern 45, 55 sind in ihren Dimensionen an die entsprechend zugeordneten Zufuhrkanäle 40, 50 angepasst. Ihre Querschnittsform und oder - fläche kann von der Querschnittsfläche und/oder Querschnittsform der Zufuhrkanäle 45, 50 abweichen, vorzugsweise ist sie größer. In einem besonderen Anwendungsfall kann es vorteilhaft sein, wenn die Querschnittsfläche und oder die Querschnittsform der Eintrittskammern 45, 55 identisch mit der Querschnittsform und/oder der Querschnittsfläche der Zufuhrkanäle 40, 50 ist. Insbesondere bilden die Eintrittskammern 45, 55 jeweils die axialen Endabschnitte der zugehörigen Zufuhrkanäle 40, 50. Die Eintrittskammern 45, 55 sind in distale Richtung jeweils durch die Umlenkflächen 41, 51 bzw. die distale Endfläche 22 der Mischkammer 11 begrenzt.

In Fig. 3 ist die parallele Anordnung der Zufuhrkanäle 30, 40, 50 gut erkennbar. Ferner ist deutlich zu sehen, dass der erste Zufuhrkanal 30 axial in die Mischkammer 11 mündet und koaxial zur Düsenöffnung 23 ausgerichtet ist. Sowohl die Eintrittskammern 45, 55, als auch die Mischkammer 11 sind vergleichsweise schmal ausgebildet. Für alle Ausführungsbeispiele gilt, dass die Länge der Mischkammer 11 möglichst klein gewählt wird, um unmittelbar vor der Düsenöffnung 23 eine kurze aber effiziente Vermischung zu ermöglichen. Vorzugsweise beträgt die Länge L der Mischkammer 11 höchstens 150 µm, insbesondere höchstens 120 µm, insbesondere höchstens 100 µm, insbesondere höchstens 70 µm, insbesondere höchstens 50 µm, insbesondere höchstens 40 µm, insbesondere höchstens 30 µm. Entsprechende Werte gelten für die Tiefe T der Eintrittsöffnungen 42, 52. Die Tiefe T der Eintrittsöffnungen 42, 52 erstreckt sich vorzugsweise über die gesamte Länge L der Mischkammer 11.

An dieser Stelle wird darauf hingewiesen, dass die Länge L der Mischkammer 11 im Wesentlichen gleichbedeutend mit der Dicke der Mischkammerplatte 10 ist, zumindest wenn die Düse eine separate, d.h. von der Düsenplatte 20 getrennte, Mischkammerplatte 10 aufweist.

Die Zufuhrkanäle 30, 40, 50 sind in einem Kanalträger 60 angeordnet bzw. in einem Kanalträger 60 gehalten. Der Kanalträger 60 umschließt zumindest abschnittsweise die Zufuhrkanäle 30, 40, 50, vorzugsweise die distalen Endabschnitte der Zufuhrkanäle 30, 40, 50. Der Kanalträger 60 kann eine zylinderförmige Außenkontur aufweisen. An einem distalen Ende des Kanalträgers 60 kann eine kreisförmige Vertiefung vorgesehen sein, so dass der Kanalträger 60 am distalen Ende eine ringförmige Brüstung 61 aufweist. Passend zu der kreisförmigen Vertiefung bzw. der ringförmigen Brüstung 61 kann die Düsenplatte 20 eine Schulter 24 aufweisen, die im montierten Zustand an der Brüstung 61 anliegt. Im Bereich der Schulter 24 weist die Düsenplatte 20 einen Außendurchmesser auf, der dem Außendurchmesser des Kanalträgers 60 entspricht. Außerhalb der Schulter 24, also in einem Mischkammerabschnitt, weist die Düsenplatte 20 hingegen einen reduzierten Außendurchmesser auf, der dem zylinderförmigen Innendurchmesser des Kanalträgers 60 im Bereich der Brüstung 61 entspricht. Somit kann die Düsenplatte 20 deckelartig, insbesondere formschlüssig, auf den Kanalträger 60 aufgesetzt werden.

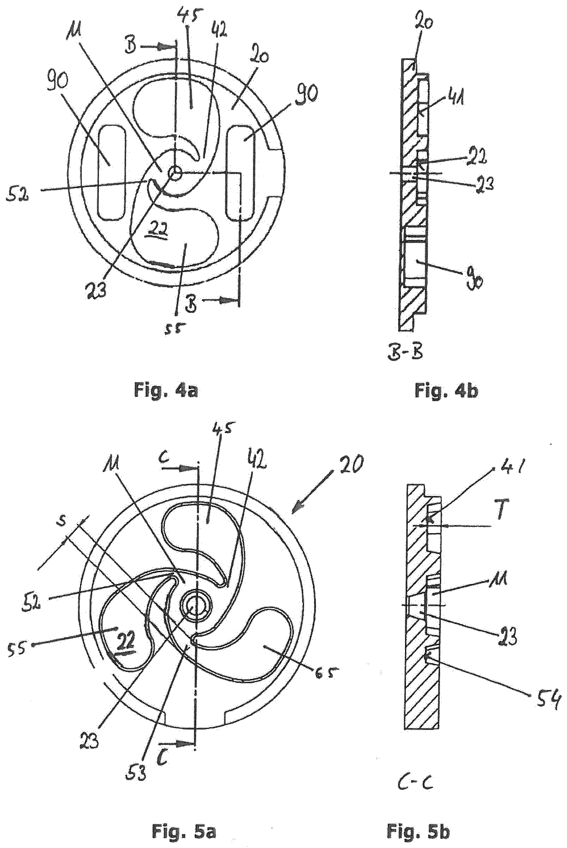

Die Fig. 4a und 4b zeigen ein weiteres Ausführungsbeispiel einer Düsenplatte 20 für eine erfindungsgemäße Düse. Die Düsenplatte 20 beinhaltet eine Mischkammer 11 sowie zwei Eintrittskammern 45, 55 mit Eintrittsöffnungen 42, 52, die in die Mischkammer 11 münden. Insbesondere münden die Eintrittsöffnungen 42, 52 tangential in die Mischkammer 11. Das bedeutet, dass die äußeren Seitenflächen 43 der Eintrittsöffnungen 42, 52 bzw. der Eintrittskammern 45, 55 kontinuierlich in die Innenumfangsfläche der Mischkammer 11 übergehen. Dies gilt für alle Ausführungsbeispiele.

Die Konstruktion und Dimensionen der Mischkammer 11 und der Eintrittskammern 45, 55 mit ihren Eintrittsöffnungen 42, 52 entsprechen im Wesentlichen der Konstruktion gemäß Fig. 2a bis 3. Zusätzlich ist bei dem Ausführungsbeispiel gemäß Fig. 4a und 4b vorgesehen, dass die Düse 1 wenigstens eine Temperierleitung (nicht dargestellt) aufweist, die in der Düsenplatte 20 in einem Temperierkanal 90 endet. Dies ermöglicht die Temperierung der Fluide, sowohl vor dem Mischvorgang wie auch während dem Mischvorgang. Die Temperierkanäle 90 sind als im Wesentlichen rechteckförmige Vertiefungen in die Düsenplatte 20 eingebracht. Die rechteckförmigen Vertiefungen können abgerundete Ecken aufweisen. Die Temperierleitung kann sich parallel zu den Zufuhrleitungen und oder den Zufuhrkanälen 30, 40, 50 vom proximalen Ende des chirurgischen Instruments bis in die Düsenplatte 20 der Düse 1 erstrecken. Dadurch ist es möglich, bereits im Bereich der Zuführung der Fluide diese zu temperieren. Eine Temperierleitung kann zwei Lumen umfassen. Einen für die Zuführung und einen für die Rückführung des Temperiermediums. Es ist auch möglich, dass zwei getrennte Temperierleitungen für die Zu- bzw. Rückführung des Mediums verwendet werden. Das chirurgische Instrument kann mehrere Temperierleitungen umfassen.

Auf diese Weise können die Zufuhrleitungen sowie die Düsenplatte 20 und somit das in der Mischkammer 11 erzeugte Fluidgemisch vor dem Austritt durch die Düsenöffnung 23 temperiert werden. Beispielsweise kann das Fluidgemisch erwärmt werden, um die Viskosität des Fluidgemischs zu reduzieren. Eine reduzierte Viskosität ist bei der Zufuhr von Zellen sinnvoll, da auf diese Weise Scherkräfte in der Mischkammer 11 reduziert werden. Eine Beschädigung der Zellen wird somit vermieden. Mit anderen Worten trägt eine Temperaturerhöhung des erzeugten Fluidgemischs und damit verbundene Viskositätsänderung zu einem schonenden Transport von Zellen bei. Ferner kann durch eine Temperaturerhöhung bewirkt werden, dass die Vernetzung von wenigstens zwei miteinander zu vermischenden Fluiden beschleunigt wird. Damit kann das Anhaften der miteinander vermischten Fluide an vorhandenem Zielgewebe positiv beeinflusst werden. Umgekehrt können die Temperierleitungen die in den Temperierkanälen 90 enden für die Kühlung der Zufuhrleitungen und der Düsenplatte 20 und folglich der Mischkammer 11 genutzt und so die Vernetzung von zwei zu vermischenden Substanzen verzögert werden. Dies kann Vorteile haben, wenn beispielsweise gewünscht ist, dass sich die Vernetzung erst bei Kontakt mit einem Zielgewebe einstellt.

Anstelle eines Temperierkanals 90 oder mehrerer Temperierkanäle 90 kann eine elektrische Heizung in die Düsenplatte 20 integriert sein.

Ein weiteres Ausführungsbeispiel einer Düsenplatte 20 für eine erfindungsgemäße Düse zeigen die Fig. 5a und 5b. Der Aufbau der Düsenplatte 20 ähnelt dem Aufbau der Düsenplatte gemäß den vorhergehenden Ausführungsbeispielen. Allerdings ist bei dieser Variante vorgesehen, dass in die Mischkammer 11 zusätzlich zum axial einmündenden ersten Zufuhrkanal 30 insgesamt drei weitere Zufuhrkanäle seitlich einmünden. Die Zufuhrkanäle weisen an ihren distalen Endabschnitten tropfenförmige Eintrittskammern 45, 55, 65 auf, wie sie bereits aus den vorhergehenden Ausführungsbeispielen bekannt sind. Der Übergang in die Mischkammer 11 erfolgt über Eintrittsöffnungen 42, 52, 53, wobei die Eintrittsöffnungen 42, 52, 53 unmittelbar vor der Düse 23 in die Mischkammer 11 münden. Konkret gelangen die Eintrittsöffnungen 42, 52, 53 an der distalen Endfläche 22 in die Mischkammer 11 bzw. weisen Umlenkflächen 41, 51, 54 auf, die ebenengleich mit der distalen Endfläche 22 ausgebildet oder durch die distale Endfläche 22 gebildet sind. Die drei Eintrittsöffnungen 42, 52, 53 weisen unterschiedliche Breiten s auf. Die Bestimmung der Mischparameter der zugeführten Fluide ist unabhängig von der Anzahl der Zufuhrleitungen bzw. Zufuhrkanäle. Die Bedingungen einer bevorzugten Vermischung der zugeführten Fluide ist oben beschrieben.

Bei dieser zusätzlich offenbarten Variante der Düse ist vorgesehen, dass die Öffnungsfläche der Eintrittsöffnungen 42, 52, 53 insbesondere die Breite s der Eintrittsöffnungen 42, 52, 53 unterschiedlich für jede Eintrittsöffnung 42, 52, 53 gewählt ist, so dass dadurch die mittlere Fließgeschwindigkeit der Fluide beim Eintritt in die Mischkammer 11 bestimmt werden kann.

Der Fluidstrahl verlässt am distalen Ende der Düsenöffnung 23 die Düse 1. Es ist möglich, dass die Zufuhrleitungen und in deren Folge die Zufuhrkanäle 30, 40, 50 jeweils mit einer gesonderten Pumpe verbindbar sind. Jede Pumpe ist vorzugsweise mit einer Durchfluss- oder einer Druckregelung versehen. Die Voraussetzungen zur Erzeugung eines kegelförmigen und oder eines punktstrahlförmiger Fluidstrahl ist oben beschrieben.

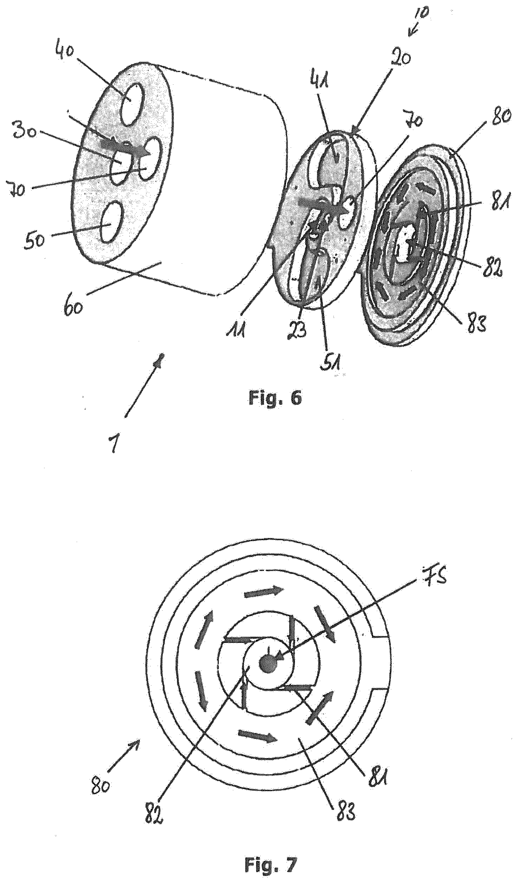

Es kann vorteilhaft sein, wenn die Düse 1 zusätzlich mit einer Vorrichtung zur Nachzerstäubung versehen ist. Eine derartig erweiterte Düse 1 zeigt Fig. 6. Bei diesem Ausführungsbeispiel ist die Düse mehrteilig ausgebildet und umfasst einen Kanalträger 60 und eine Düsenplatte 20, in die die Mischkammer 11 integriert ist. Die Düsenplatte 20 weist also einen Mischkammerabschnitt auf bzw. ist integral, d.h. einteilig, mit der Mischkammerplatte 10 ausgebildet. Der Aufbau der Düsenplatte 20 sowie des Kanalträgers 60 entspricht im Wesentlichen dem Aufbau bei den vorhergehenden Ausführungsbeispielen. Hinzu kommt bei dem Ausführungsbeispiel gemäß Fig. 6 ein Gaszufuhrkanal 70, der in der Düse 1 ausgebildet ist.

Der Gaszufuhrkanal 70 verläuft parallel zu den Zufuhrkanälen 30, 40, 50. Der Gaszufuhrkanal 70 erstreckt sich durch den Kanalträger 60 und die Düsenplatte 20 bzw. durchdringt beide. Folglich verläuft der Gaszufuhrkanal 70 parallel zur Mischkammer 11 und/oder zur Düsenöffnung 23. An die Düsenplatte 20 anschließend ist eine Zerstäuberplatte 80 vorgesehen. Die Zerstäuberplatte 80 weist eine zentrale Zerstäuberöffnung 82 auf, die mit der Düsenöffnung 23 fluchtet. Die Zerstäuberöffnung 82 ist koaxial zur Düsenöffnung 23 angeordnet. Die Zerstäuberöffnung 82 ist ebenfalls koaxial zur Mischkammer 11 und zum ersten Zufuhrkanal 30 angeordnet. Um die Zerstäuberöffnung 82 verläuft eine Ringnut 83, die als ringförmige Vertiefung auf einer proximalen Seite der Zerstäuberplatte 80 angeordnet ist. Die Ringnut 83 steht in Fluidverbindung mit dem Gaszufuhrkanal 70. Das über den Gaszufuhrkanal 70 einströmende Gas wird gleichmäßig über die Ringnut 83 verteilt.

Zwischen der Ringnut 83 und der Zerstäuberöffnung 82 sind Zerstäuberkanäle 81 angeordnet. Die Zerstäuberkanäle 81 münden tangential in die kreisförmige Zerstäuberöffnung 82. Die Zerstäuberkanäle 81 und die Ringnut 83 werden auf drei Seiten durch Elemente der Zerstäuberplatte 80 begrenzt. Eine vierte Seite des Zerstäuberkanals 81 und der Ringnut 83 wird im montierten Zustand durch eine distale Außenfläche der Düsenplatte 20 begrenzt.

In einer anderen Ausführung der erweiterten Düse (nicht dargestellt) kann die distale Endfläche der Düsenplatte 20 die Ringnut 83 und die Zerstäuberkanäle 81 umfassen. Die oben beschriebenen Merkmale der Zerstäuberplatte 80 sind in die Düsenplatte 20 integriert. Die Zerstäuberkanäle 81 sowie die Ringnut 83 werden dann auf drei Seiten durch Elemente der Düsenplatte 20 begrenzt. Die Zerstäuberplatte 80 enthält in diesem Fall nur die Zerstäuberöffnung 82. Die proximale Fläche der Zerstäuberplatte 80 bildet dann einen Deckel der Ringnut 83 und der Zerstäuberkanäle 81 und schließt diese distal ab.

Die Querschnittsfläche eines Zerstäuberkanals 81 ist bedeutend geringer als die Querschnittsfläche der Ringnut 83. Sogar die Summe der Querschnittsflächen der Zerstäuberkanäle 81 ist bedeutend geringer als die Querschnittsfläche der Ringnut 83. Dies ermöglicht eine gleichmäßige Gaszufuhr bzw. einen gleichförmigen Gasaustritt aus der Zerstäuberöffnung 82 obwohl nur ein Gaszufuhrkanal 70 in der Düse 1 angeordnet ist. Aufgrund der großen Querschnittsfläche der Ringnut 83 im Vergleich zu der kleinen Gesamtquerschnittsfläche der Zerstäuberkanäle 81 verteilt sich das zugeführte Gas gleichmäßig in der Ringnut 83, unabhängig der Anordnung des Gaszufuhrkanals 70. Mit anderen Worten, der Gaszufuhrkanal 70 muss nicht koaxial an der Düse 1 angeordnet sein, sondern kann radial nach Außen versetzt angeordnet sein und trotzdem ist ein gleichmäßiger Gasaustritt aus der Zerstäuberöffnung 82 gewährleistet.

Zum anderen wird durch die Reduktion des Strömungsquerschnitts beim Übergang von der Ringnut 83 in die Zerstäuberkanäle 81 das Gas vor dem Eintritt in die Zerstäuberöffnung 82 beschleunigt. Das beschleunigte Gas trifft auf den Fluidstrahl FS, der vorzugsweise die Form eines Kegelstrahls aufweist. Somit werden die bereits durch den Kegelstahl gebildeten Tröpfchen im Fluidstrahl FS weiter zerteilt, so dass sich im Wesentlichen ein Aerosol bildet. Überdies ermöglicht die tangentiale Gaszufuhr eine Beeinflussung des Sprühwinkels. Konkret kann der Kegelstrahl weiter aufgeweitet werden. Für die Funktionsfähigkeit der Zerstäuberplatte 80 ist es zweckmäßig, wenn, wie in Fig. 6 und 7 gezeigt ist, die Zerstäuberöffnung 82 einen größeren Querschnittsdurchmesser als die Düsenöffnung 23 aufweist.

Damit die oben beschriebenen Vorteile ihre Wirkung vollständig entfalten können sind mehrere Zerstäuberkanäle erforderlich. Bei dem Ausführungsbeispiel gemäß Fig. 6 und 7 sind vier Zerstäuberkanäle 81 vorgesehen. Das Gas, das über den Gaszufuhrkanal 70 und die Ringnut 83 in die Zerstäuberkanäle 81 strömt, verlässt die Zerstäuberkanäle 81 im Bereich der Zerstäuberöffnung 82. Somit entsteht um den Fluidstrahl FS eine im Wesentlichen zylinderförmig ausgebildete Gasströmung, die den Fluidstrahl FS umgibt.

Wie zuvor erläutert, wird mit der erfindungsgemäßen Düse, die vorzugsweise Teil eines Wasserstrahlapplikators ist, ein Transfer von Zellen mit relativ hoher Überlebensrate ermöglicht. Die Düse gewährleistet also einen schonenden Transport bzw. eine schonende Applikation von Zellen. Dazu ist es angedacht, die Zellen über den ersten Zufuhrkanal 30 in die Mischkammer 11 zu führen, wobei der erste Zufuhrkanal 30 axial in die Mischkammer 11 mündet und koaxial zur Düsenöffnung 23 ausgerichtet ist. So erfahren die im ersten Fluidstrahl mitgeführten Zellen keine Umlenkung, die zum Auftreten von Scherkräften und zur Beschädigung der Zellen beitragen könnte. Durch den geradlinigen Strömungsverlauf werden die Zellen also geschont. Die Zellen können separat über den ersten Zufuhrkanal 30 appliziert werden. Vorzugsweise ist allerdings vorgesehen, ein Trägerfluid in der Mischkammer 11 beizumischen. Das Trägerfluid kann beispielsweise medizinisches Wasser, organische Substanzen wie beispielsweise Blutplasma, Blutserum oder organische Kleber, biologische Substanzen wie Enzyme, Gerinnungsfaktoren und/oder weitere Blutbestandteile, Lösungen, Suspensionen, Emulsionen mit medizinischen und/oder pharmazeutischen Bestandteilen oder eine Kochsalzlösung sein. Auf diese Weise wird im Wesentlichen eine Düse für einen Wasserstrahlapplikator bereitgestellt, der einen schonenden Zelltransport ermöglicht. Mittels der Wasserstrahltechnologie kann der Zellentransport druckbegrenzt, zielgenau und gut mengengesteuert werden.

Ein wesentlicher Aspekt der Erfindung besteht darin, dass die weiteren Zufuhrkanäle, insbesondere der zweite und dritte Zufuhrkanal 40, 50, nicht nur seitlich in die Mischkammer 11 münden, sondern der Querschnittsdurchmesser bzw. die Querschnittsdimension des zweiten und/oder dritten Zufuhrkanals 40, 50 bis zum Eintritt in die Mischkammer 11 reduziert wird. Damit wird erreicht, dass sich die Strömungsgeschwindigkeit des im jeweiligen Zufuhrkanal 40, 50 geführten Fluids erhöht. In der Folge sinkt der statische Druck der zugeführten Fluide. Insofern bildet die Eintrittskammer 45, 55 und die Eintrittsöffnung 42, 52 jeweils eine Beschleunigungsstrecke für das im Zufuhrkanal 40, 50 geführte Fluid. Zum schonenden Zelltransport trägt ferner bei, dass die einzelnen Kanäle, insbesondere der erste Zufuhrkanal 30, der zweite Zufuhrkanal 40 und der dritte Zufuhrkanal 50, gemeinsam in die Mischkammer 11 geführt werden. Die Vermischung der einzelnen Fluide erfolgt in der Mischkammer 11. In der Folge davon ist die Strecke, in der das Zellen führende Fluid Scherkräften bzw. Mischungskräften ausgesetzt ist, äußerst gering, was die Zellen schont. Zudem wird wegen dieser kurzen Strecke in der Düse bzw. der kurzen Zeit in der die Fluide in der Düse vermischt werden auch die Vernetzung der unterschiedlichen Substanzen weitestgehend, im Idealfall vollständig unterdrückt. Eine erfindungsgemäße Düse ist klein und kompakt ausgebildet. Die Vernetzung der vermischten Fluide findet hauptsächlich außerhalb der Düse statt. Dadurch wird ein Verstopfen der Düse weitestgehend vermieden. Zusätzlich wird das Ansteigen der Scherkräfte in Folge der Vernetzung vermieden. Entsprechendes gilt für die Anordnung der Düsenöffnung 23, die unmittelbar nach der Mischkammer 11 angeordnet ist. Konkret wird dies erfindungsgemäß dadurch erreicht, dass die seitlichen Eintrittsöffnungen 42, 52 direkt an die Fläche angrenzen, aus der auch die Düsenöffnung 23 entspringt. In diesem Ausführungsbeispiel entspricht dies der distalen Endfläche 22 der Mischkammer 11.

Die Anwendungsmöglichkeiten der erfindungsgemäßen Düse sind vielfältig. Beispielsweise können mit der erfindungsgemäßen Düse Zellen an Organen des Gastrointestinaltrakts, beispielsweise den Magen, den Darm oder den Ösophagus geführt werden. Grundsätzlich weisen Organe, insbesondere die Außenwände von Organen einen Gewebeaufbau aus mehreren, zum Teil unterschiedlichen, aufeinanderliegenden Zellschichten auf. Die innere Organwand umfasst insbesondere die Gewebezellschichten der Mukosa, der Submukosa, der Muscularis und/oder der Serosa. Den einzelnen Zellschichten kommen unterschiedliche funktionelle Aufgaben zu. Um einzelne geschädigte Stellen in der Organwand zu behandeln, sind also unterschiedliche Zellarten zuzuführen.

Mit der erfindungsgemäßen Düse, die im Wesentlichen als Mehrstoffdüse ausgebildet ist, kann die Behandlung derartiger Organwände besonders effizient durchgeführt werden. Da die erfindungsgemäße Düse mehrere Zufuhrkanäle 30, 40, 50 aufweist, können unterschiedliche Substanzen und/oder unterschiedliche Fluidgemische während eines Behandlungsvorganges über die Düsenöffnung appliziert werden. Die einzelnen Zufuhrkanäle 30, 40, 50 können unabhängig voneinander aktiviert werden. So können zu unterschiedlichen Zeitpunkten während der medizinischen Behandlung unterschiedliche Substanzen oder Gemische aus unterschiedlichen Substanzen zugeführt werden.

Ein chirurgisches Instrument mit einer erfindungsgemäßen Düse kann ferner mit einem chirurgischen Gerät verbunden oder verbindbar sein. Das chirurgische Gerät kann eine Steuerung bzw. Regelung aufweisen, mit der unterschiedliche Fluidzufuhrdrücke oder unterschiedliche Fluidvolumenströme einstellbar sind. Vorzugsweise wird zum Versprühen, insbesondere für den Kegelstrahlbetrieb, ein Fluidzufuhrdruck im Bereich von 2,5 bis 60 bar, insbesondere im Bereich von 5 bar bis 40 bar eingestellt. Zur Applikation eines Aerosols oder eines Aerosol-Plasmas ist es vorteilhaft, wenn die Fluidzufuhrdrücke zwischen 2 bar und 10 bar eingestellt werden. Bevorzugte Volumenströme für das zugeführte Fluid in den Zufuhrkanälen 30, 40, 50 zum Versprühen sind im Bereich von 5 ml/min bis 40 ml/min, insbesondere im Bereich von 10 ml/min bis 30 ml/min, vorzugsweise im Bereich von 15 ml/min bis 25 ml/min. Für die nadellose Injektion von Flüssigkeiten bzw. Fluiden ist es vorteilhaft, wenn der Volumenstrom in wenigstens einem der Zufuhrkanäle 30, 40, 50 zwischen 5 ml/min und 110 ml/min, insbesondere zwischen 10 ml/min und 100 ml/min, insbesondere zwischen 20 ml/min und 90 ml/min eingestellt wird. Für die Zufuhr von Aerosol-Plasma hat es sich als vorteilhaft erwiesen, wenn der Volumenstrom in wenigstens einem der Zufuhrkanäle 30, 40, 50 0,2 ml/min bis 5 ml/min, insbesondere 0,3 ml/min bis 3 ml/min, vorzugsweise 0,5 ml/min bis 1 ml/min beträgt.

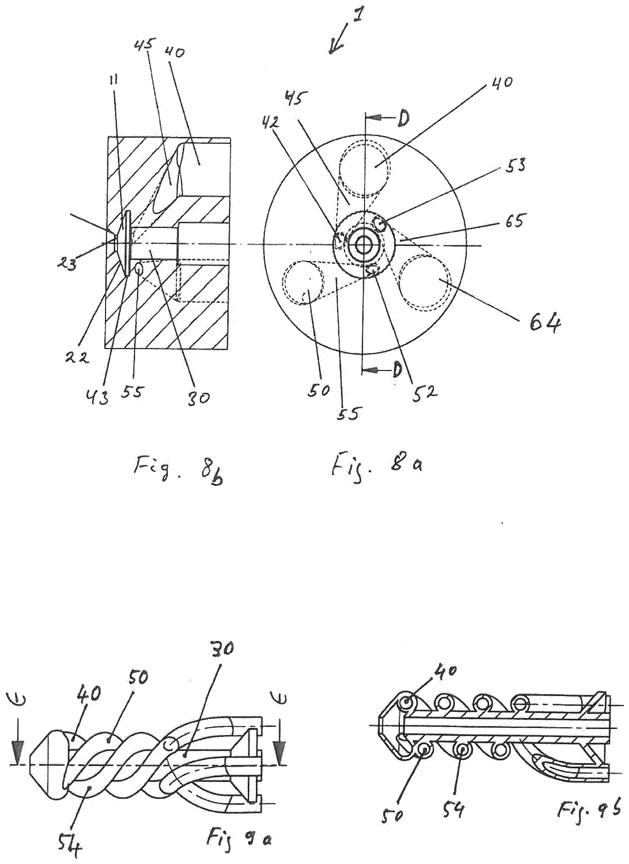

Figur 8 zeigt ein Ausführungsbeispiel bei der die Düse 1 einteilig ausgeführt ist. Dabei sind die Düsenöffnung 23, die Mischkammer 11, die Eintrittskammern 45, 55, 65, die Eintrittsöffnungen 42, 52, 53 sowie die Zufuhrkanäle 30, 40, 50, 64 integral in einem Bauteil ausgeführt. Erfindungsgemäß fließt bei dieser Ausführungsform der Düse 1 das Fluid durch kantenfreie Umlenkungen bis in die Mischkammer. Die radial nach außen versetzten Zufuhrkanäle 40, 50, 64, sind erfindungsgemäß rohrförmig gebildet. Im Unterschied zu den oben beschriebenen Ausführungsbeispielen, bei denen die Zufuhrkanäle 40, 50, insbesondere deren Umlenkbereiche durch aneinandergefügte Flächenabschnitte gebildet sind, sind bei der Ausführungsform gemäß Figur 8 diese Umlenkbereiche durch zylinderförmige , kantenfreie Leitungsabschnitte gebildet. Dies ermöglicht, auch bei Änderung der Fließrichtung um bis zu 90 Grad eine graduelle Umlenkung des zuzuführenden Fluids. Dadurch kann das zuzuführende Fluid schonend der Mischkammer 11 zugeführt werden. Die Ausgestaltung der Zufuhrkanäle 40, 50, 64 ermöglicht eine kontinuierliche, graduelle Umlenkung der Strömungsrichtung der durch diese Zufuhrkanäle strömenden Fluide vom proximalen Ende der Düse 1 bis zum Eintritt in die Mischkammer 11. Obwohl sich die Strömungsrichtung ausgehend vom distalen Ende der Zufuhrkanäle 40, 50, 64 bis in die Mischkammer um bis zu 90 Grad ändert, weisen die Zufuhrkanäle auf dieser gesamten Strecke keine Stellen auf, die eine abrupte Umlenkung der Strömungsrichtung erzeugen. Die Zufuhrkanäle sind ausschließlich mit Radien versehen, scharfkantige Ecken werden vermieden. Erfindungsgemäß weisen die Zufuhrkanäle auf ihrem Weg bis in die Mischkammer 11 Winkel auf, die kleiner als 90 Grad, vorzugsweise zwischen 40 und 65 Grad, insbesondere vorzugsweise zwischen 10 und 30 Grad betragen.

Die Ausgestaltung der Zufuhrkanäle 40, 50 und 64 wird beispielhaft am Zufuhrkanal 40 beschrieben und gilt auch für die Zufuhrkanäle 50, 64 und weitere wenn vorhanden. Der Zufuhrkanal 40 reicht vom proximalen Ende der Düse 1 bis in die Mischkammer 11. Er umfasst die Eintrittskammer 45 und die Eintrittsöffnung 42. Die Eintrittskammer 45, welche die Eintrittsöffnung 42 umfasst ist in Form eines schiefen Kreiskegelstumpfs ausgebildet. Der Kegelstumpft verjüngt sich in Richtung der Eintrittsöffnung 42. Dieser Kegelstumpf mündet tangential im Bereich der Eintrittsöffnung 42 unter einem Winkel, in Bezug auf die Längsachse der Düse 1 in die Mischkammer 11. Dabei ist die Eintrittsöffnung 42 des Zufuhrkanals 40 in Bezug zu dem Zufuhrkanal 30 bzw. zu der Düsenöffnung 23 radial nach außen versetzt. Die Eintrittsöffnung 42 geht unter strömungsgünstigen Bedingungen in die Mischkammer 11 über. Dieser Übergang kann beispielsweise in der proximalen Endfläche 21 der Mischkammer 11 enden. Er kann aber auch in die äußere Seitenfläche 43 der Mischkammer 11 unter strömungsgünstigen Bedingungen übergehen. Dabei kann der Durchmesser der Eintrittsöffnung 42 größer sein als die Breite der Seitenfläche 43. Dies hat dann zur Folge, dass die Eintrittsöffnung 42 teilweise in der proximalen Endfläche 22, teilweise in der Seitenfläche 43 und möglicherweise teilweise in der distalen Endfläche 21 der Mischkammer 11 übergeht. Die Schwerpunktlinie (Mittellinie) der Eintrittskammer 45 ist in einem Winkel zur proximalen Endfläche 21 der Mischkammer 11 angeordnet. Dieser Winkel ist kleiner als 90 Grad, vorzugsweise maximal 65 Grad, vorzugsweise maximal 50 Grad. Beim Übergang der Eintrittskammer 45 in die proximale Endfläche 21 der Mischkammer 11 ist der Verlauf der Schwerpunktlinie der Eintrittskammer 45 so angeordnet, dass eine senkrechte Projektion dieser Schwerpunktlinie auf die proximale Endfläche 21 tangential in einen zur Düsenöffnung 23 konzentrisch angeordneten Kreis übergeht. Die Ausführung der Eintrittskammer 45 als ein Teil des Zufuhrkanals mit sich gleichmäßig verengendem Querschnitt bewirkt eine Erhöhung der Strömungsgeschwindigkeit des durch den Zufuhrkanal 40, fließenden Fluids bis zum Eintritt in die Mischkammer 11. Die in den Ausführungsbeispielen gemäß Figur 1 bis 7 beschriebenen Merkmale einer Mischkammer 11 und der Düsenöffnung 23 gelten auch für die Mischkammer 11 und die Düsenöffnung 23 gemäß Figur 8.

Die erfindungsgemäße Gestaltung und Anordnung des Zufuhrkanal 40 hat auf das zugeführte Fluid den Vorteil, dass die graduellen Umlenkungen zu einem geringen Druckabfall im Fluid führen. Dies ermöglicht einen niedrigen Förderdruck was die schonende Zuführung des Fluids ermöglicht. Durch die erfindungsgemäßen graduellen Umlenkungen des Zufuhrkanals werden die bei Richtungsänderungen eines Fluids erzeugten Impulsänderungskräfte auf das Fluid reduziert was zusätzlich den schonenden Transport des Fluids bis in die Mischkammer 11 ermöglicht.

Figur 9a und 9b zeigen ein erfindungsgemäßes Ausführungbeispiel mit denselben strukturellen Merkmalen und denselben Vorteilen wie das Ausführungsbeispiel gemäß Figur 8. Im Unterschied zum Ausführungsbeispiel gemäß Figur 8 sind die Zufuhrkanäle 40, 50, 64 beim Ausführungsbeispiel gemäß Figur 9 durch rohrförmige Leitungen gebildet. Figur 9a und 9b zeigen auch eine einstückig ausgebildete Düse 1 deren Strömungseigenschaften der zugeführten Fluide mit den Strömungseigenschaften eines Ausführungsbeispiels gemäß Figur 8 identisch sind.

Einstückige mehrlumige Düsen können beispielsweise durch Laser-Sinterverfahren hergestellt werden.

Die Erfindung betrifft eine Düse zur Zufuhr von biologischem Material, insbesondere Zellen, mit
- einer Mischkammer (11), die durch eine proximale Endfläche (21) und eine von der proximalen Endfläche (21) beabstandete distale Endfläche (22) begrenzt ist,
- wenigstens einer Düsenöffnung (23), die in der distalen Endfläche (22) ausgebildet ist, und
- wenigstens zwei Zufuhrkanälen (30, 40, 50), die in die Mischkammer (11) münden.

Die Erfindung zeichnet sich dadurch aus, dass ein erster Zufuhrkanal (30) in der proximalen Endfläche (21) angeordnet ist und koaxial zur Düsenöffnung (23) in die Mischkammer (11) mündet und ein zweiter Zufuhrkanal (40) eine Eintrittsöffnung (42) aufweist, die an der distalen Endfläche (22) seitlich, insbesondere tangential, in die Mischkammer (11) mündet.

### Bezugszeichenliste

- 1: Düse
- 10: Mischkammerplatte
- 11: Mischkammer
- 12: Innenumfangsfläche

- 20: Düsenplatte
- 21: proximale Endfläche
- 22: distale Endfläche
- 23: Düsenöffnung
- 24: Schulter

- 30: erster Zufuhrkanal
- 40: zweiter Zufuhrkanal
- 41, 51, 54: Umlenkfläche
- 42, 52, 53: Eintrittsöffnung
- 43: äußere Seitenfläche
- 44: innere Seitenfläche
- 45, 55, 65: Eintrittskammer
- 46,: Steg
- 50: dritter Zufuhrkanal
- 64: weiterer Zufuhrkanal
- 60: Kanalträger
- 61: Brüstung

- 70: Gaszufuhrkanal

- 80: Zerstäuberplatte
- 81: Zerstäuberkanal
- 82: Zerstäuberöffnung
- 83: Ringnut

- 90: Temperierkanal

- s: Breite der Eintrittsöffnung
- s1: Breite der ersten Eintrittsöffnung 42
- s2: Breite der zweiten Eintrittsöffnung 52
- T: Tiefe der Eintrittsöffnung 42, 52
- L: Länge der Mischkammer 11
- FS: Fluidstrahl

## Patentansprüche

1. Düse für ein medizinisches Instrument
zur Zufuhr von biologischem Material mit
- einer Mischkammer (11), die durch eine proximale Endfläche (21) und eine von der proximalen Endfläche (21) beabstandete distale Endfläche (22) begrenzt ist,
- wenigstens einer Düsenöffnung (23), die in der distalen Endfläche (22) ausgebildet ist, und
- wenigstens zwei Zufuhrkanälen (30, 40, 50), die in die Mischkammer (11) münden,
wobei
ein erster Zufuhrkanal (30) in der proximalen Endfläche (21) angeordnet ist und koaxial zur Düsenöffnung (23) in die Mischkammer (11) mündet,
**dadurch gekennzeichnet, dass** ein
zweiter Zufuhrkanal (40) eine Eintrittsöffnung (42) aufweist, die an der distalen Endfläche (22) tangential in die Mischkammer (11) mündet.

2. Düse nach Anspruch 1,
**dadurch gekennzeichnet, dass**
die Mischkammer (11) eine Länge L aufweist, die höchstens 500 µm, insbesondere höchstens 250 µm, insbesondere höchstens 150 µm, insbesondere höchstens 100 µm, insbesondere höchstens 75 µm,
insbesondere höchstens 50 µm, insbesondere höchstens 30 µm, beträgt.

3. Düse nach Anspruch 1 oder 2,
**dadurch gekennzeichnet, dass**
die seitliche Eintrittsöffnung (42) eine Tiefe T aufweist, die dem Abstand zwischen der proximalen Endfläche (21) und der distalen Endfläche (22) entspricht.

4. Düse nach einem der vorhergehenden Ansprüche,
**dadurch gekennzeichnet, dass**
die distale Endfläche (22) der Mischkammer (11) senkrecht zu einer Längsachse des zweiten Zufuhrkanals (40) angeordnet ist.

5. Düse nach einem der vorhergehenden Ansprüche,
**dadurch gekennzeichnet, dass**
die seitliche Eintrittsöffnung (42) ein rechteckiges Querschnittsprofil aufweist und/oder sich in Richtung zur Mischkammer (11) verjüngt.

6. Düse nach Anspruch 5 **dadurch gekennzeichnet, dass** die seitliche Eintrittsöffnung (42) insbesondere zwei gekrümmte Seitenflächen (43, 44) aufweist, die zur Mischkammer (11) konvergieren.

7. Düse nach Anspruch 6,
**dadurch gekennzeichnet, dass**
eine äußere Seitenfläche (43) der seitlichen Eintrittsöffnung (42) kontinuierlich in eine Innenumfangsfläche (12) der Mischkammer (11) übergeht.

8. Düse nach einem der vorhergehenden Ansprüche,
**dadurch gekennzeichnet, dass**
wenigstens ein dritter Zufuhrkanal (50) mit einer weiteren seitlichen Eintrittsöffnung (52) vorgesehen ist, die an der distalen Endfläche (22) seitlich in die Mischkammer (11) mündet.

9. Düse nach einem der vorhergehenden Ansprüche,
**dadurch gekennzeichnet, dass**
die Zufuhrkanäle (30, 40, 50) und/oder die Mischkammer (11) und/oder die Düsenöffnung (23) einteilig oder mehrteilig ausgebildet sind.

10. Düse nach einem der vorhergehenden Ansprüche,
**dadurch gekennzeichnet, dass**
die Zufuhrkanäle (30, 40, 50) in einem Kanalträger (60) und/oder die Düsenöffnung (23) in einer Düsenplatte (20) ausgebildet ist, wobei die Mischkammer (11) insbesondere in der Düsenplatte (20) oder in einer Mischkammerplatte (10) ausgebildet ist, die zwischen der Düsenplatte (20) und dem Kanalträger (60) angeordnet ist.

11. Düse nach einem der vorhergehenden Ansprüche,
**dadurch gekennzeichnet, dass**
wenigstens ein Temperierkanal (90) vorgesehen ist derart, dass ein durch wenigstens einen Zufuhrkanal (30, 40, 50) strömendes Fluid temperierbar ist, wobei der Temperierkanal (90) insbesondere eine Zuleitung und eine Rückleitung verbindet derart, dass ein geschlossener Temperierkreislauf gebildet ist.

12. Düse nach einem der vorhergehenden Ansprüche,
**dadurch gekennzeichnet, dass**
wenigstens eine Zerstäuberplatte (80) vorgesehen ist, die distal mit der Düsenplatte (20) verbunden ist, und eine koaxial zur Düsenöffnung (23) angeordnete Zerstäuberöffnung (82) aufweist, wobei wenigstens ein Zerstäuberkanal (81) in der Zerstäuberplatte (80) ausgebildet ist, der die Zerstäuberöffnung (82) mit einem Gaszufuhrkanal (70) verbindet und vorzugsweise tangential in die Zerstäuberöffnung (82) mündet.

13. Düse nach einem der vorhergehenden Ansprüche,
**dadurch gekennzeichnet, dass**
die Querschnittsfläche der Eintrittsöffnung (42, 52) kleiner als die Querschnittsfläche der Zuleitung (30) ausgebildet ist.

14. Verfahren zum Mischen von wenigstens zwei Fluiden mittels einer Düse für medizinische Zwecke nach Anspruch 13, bei dem die Fluide mit unterschiedlichen Volumenströmen in die Mischkammer (11) einströmen, wobei das Verhältnis der Querschnittsflächen der Eintrittsöffnungen (42, 52) dem Verhältnis der Volumenströme entspricht, so dass die Fluide mit im Wesentlichen gleicher mittlerer Eintrittsgeschwindigkeit in die Mischkammer (11) einströmen.

15. Medizinische Vorrichtung, insbesondere chirurgisches Instrument, mit einer Düse nach einem der vorhergehenden Ansprüche.

## Claims

1. Nozzle for a medical instrument for feeding biological material, comprising
- a mixing chamber (11) which is delimited by a proximal end surface (21) and a distal end surface (22) which is spaced apart from the proximal end surface (21),
- at least one nozzle opening (23) which is formed in the distal end surface (22), and
- at least two feed ducts (30, 40, 50) which open into the mixing chamber (11),
wherein a first feed duct (30) is arranged in the proximal end surface (21) and opens, coaxially to the nozzle opening (23), into the mixing chamber (11), **characterized in that**
a second feed duct (40) has an inlet opening (42) which opens tangentially into the mixing chamber (11) at the distal end surface (22).

2. Nozzle according to Claim 1,
**characterized in that**
the mixing chamber (11) has a length L, which is at most 500 *µ*m, in particular at most 250 *µ*m, in particular at most 150 *µ*m, in particular at most 100 *µ*m, in particular at most 75 *µ*m, in particular at most 50 *µ*m, and in particular at most 30 *µ*m.

3. Nozzle according to Claim 1 or 2,
**characterized in that**
the lateral inlet opening (42) has a depth T which corresponds to the distance between the proximal end surface (21) and the distal end surface (22).

4. Nozzle according to one of the preceding claims, **characterized in that**
the distal end surface (22) of the mixing chamber (11) is arranged perpendicularly to a longitudinal axis of the second feed duct (40).

5. Nozzle according to one of the preceding claims, **characterized in that**
the lateral inlet opening (42) has a rectangular cross-sectional profile and/or tapers in the direction of the mixing chamber (11).

6. Nozzle according to Claim 5,
**characterized in that**
the lateral inlet opening (42) has in particular two curved lateral surfaces (43, 44) which converge towards the mixing chamber (11).

7. Nozzle according to Claim 6,
**characterized in that**
an outer lateral surface (43) of the lateral inlet opening (42) merges continuously into an inner peripheral surface (12) of the mixing chamber (11).

8. Nozzle according to one of the preceding claims, **characterized in that**
at least a third feed duct (50) having a further lateral inlet opening (52) is provided, which inlet opening opens laterally into the mixing chamber (11) at the distal end surface (22).

9. Nozzle according to one of the preceding claims, **characterized in that**
the feed ducts (30, 40, 50) and/or the mixing chamber (11) and/or the nozzle opening (23) are or is of one-part or multi-part design.

10. Nozzle according to one of the preceding claims, **characterized in that**
the feed ducts (30, 40, 50) are formed in a duct carrier (60) and/or the nozzle opening (23) is formed in a nozzle plate (20), wherein the mixing chamber (11) is formed in particular in the nozzle plate (20) or in a mixing chamber plate (10) which is arranged between the nozzle plate (20) and the duct carrier (60).

11. Nozzle according to one of the preceding claims, **characterized in that**
at least one temperature-control duct (90) is provided in such a way that a fluid flowing through at least one feed duct (30, 40, 50) can be temperature-controlled, wherein the temperature-control duct (90) in particular connects a supply line and a return line in such a way that a closed temperature-control circuit is formed.

12. Nozzle according to one of the preceding claims, **characterized in that**
at least one atomizer plate (80) is provided which is connected distally to the nozzle plate (20) and has an atomizer opening (82) arranged coaxially to the nozzle opening (23), wherein at least one atomizer duct (81) is formed in the atomizer plate (80), connects the atomizer opening (82) to a gas supply duct (70) and preferably opens tangentially into the atomizer opening (82).

13. Nozzle according to one of the preceding claims, **characterized in that**
the cross-sectional area of the inlet opening (42, 52) is designed to be smaller than the cross-sectional area of the supply line (30).

14. Method for mixing at least two fluids by means of a nozzle for medical purposes according to Claim 13, in which method the fluids flow into the mixing chamber (11) with different volumetric flows, wherein the ratio of the cross-sectional areas of the inlet openings (42, 52) corresponds to the ratio of the volumetric flows, with the result that the fluids flow into the mixing chamber (11) with a substantially identical average inlet velocity.

15. Medical device, in particular a surgical instrument, having a nozzle according to one of the preceding claims.

## Revendications

1. Buse destinée à un instrument médical pour l'alimentation de matériau biologique, avec
- une chambre de mélange (11), qui est limitée par une face d'extrémité proximale (21) et une face d'extrémité distale (22) espacée de la face d'extrémité proximale (21),
- au moins un orifice de buse (23), qui est formé dans la face d'extrémité distale (22), et
- au moins deux canaux d'alimentation (30, 40, 50), qui débouchent dans la chambre de mélange (11),
dans laquelle un premier canal d'alimentation (30) est disposé dans la face d'extrémité proximale (21) et débouche dans la chambre de mélange (11) coaxialement à l'orifice de buse (23),
**caractérisée en ce qu'**un deuxième canal d'alimentation (40) présente un orifice d'entrée (42), qui débouche dans la chambre de mélange (11) tangentiellement à la face d'extrémité distale (22).

2. Buse selon la revendication 1, **caractérisée en ce que** la chambre de mélange (11) présente une longueur L, qui vaut au maximum 500 *µ*m, en particulier au maximum 250 *µ*m, en particulier au maximum 150 *µ*m, en particulier au maximum 100 *µ*m, en particulier au maximum 75 *µ*m, en particulier au maximum 50 *µ*m, en particulier au maximum 30 *µ*m.

3. Buse selon la revendication 1 ou 2, **caractérisée en ce que** l'orifice d'entrée latéral (42) présente une profondeur T, qui correspond à la distance entre la face d'extrémité proximale (21) et la face d'extrémité distale (22).

4. Buse selon l'une quelconque des revendications précédentes, **caractérisée en ce que** la face d'extrémité distale (22) de la chambre de mélange (11) est disposée perpendiculairement à un axe longitudinal du deuxième canal d'alimentation (40).

5. Buse selon l'une quelconque des revendications précédentes, **caractérisée en ce que** l'orifice d'entrée latéral (42) présente un profil de section transversale rectangulaire et/ou se rétrécit en direction de la chambre de mélange (11).

6. Buse selon la revendication 5, **caractérisée en ce que** l'orifice d'entrée latéral (42) présente au moins deux faces latérales courbes (43, 44), qui convergent vers la chambre de mélange (11).

7. Buse selon la revendication 6, **caractérisée en ce qu'**une face latérale extérieure (43) de l'orifice d'entrée latéral (42) se prolonge de façon continue dans une face périphérique intérieure (12) de la chambre de mélange (11).

8. Buse selon l'une quelconque des revendications précédentes, **caractérisée en ce qu'**il est prévu au moins un troisième canal d'alimentation (50) avec un autre orifice d'entrée latéral (52), qui débouche latéralement dans la chambre de mélange (11) sur la face d'extrémité distale (22).

9. Buse selon l'une quelconque des revendications précédentes, **caractérisée en ce que** les canaux d'alimentation (30, 40, 50) et/ou la chambre de mélange (11) et/ou l'orifice de buse (23) sont réalisés en une seule partie ou en plusieurs parties.

10. Buse selon l'une quelconque des revendications précédentes, **caractérisée en ce que** les canaux d'alimentation (30, 40, 50) sont réalisés dans un porte-canaux (60) et/ou l'orifice de buse (23) est formé dans une plaque de buse (20), dans laquelle la chambre de mélange (11) est formée en particulier dans la plaque de buse (20) ou dans une plaque de chambre de mélange (10), qui est disposée entre la plaque de buse (20) et le porte-canaux (60).

11. Buse selon l'une quelconque des revendications précédentes, **caractérisée en ce qu'**il est prévu au moins un canal de conditionnement thermique (90), de telle manière qu'un fluide circulant à travers ledit au moins un canal d'alimentation (30, 40, 50) puisse être conditionné thermiquement, dans laquelle le canal de conditionnement thermique (90) relie au moins une conduite d'arrivée et une conduite de retour, de telle manière qu'un circuit fermé de conditionnement thermique soit formé.

12. Buse selon l'une quelconque des revendications précédentes, **caractérisée en ce qu'**il est prévu au moins une plaque de pulvérisateur (80), qui est reliée en position distale à la plaque de buse (20), et présente un orifice de pulvérisation (82) disposé coaxialement à l'orifice de buse (23), dans laquelle au moins un canal de pulvérisateur (81) est formé dans la plaque de pulvérisateur (80) et relie l'orifice de pulvérisateur (82) à un canal d'alimentation en gaz (70) et débouche de préférence tangentiellement dans l'orifice de pulvérisateur (82).

13. Buse selon l'une quelconque des revendications précédentes, **caractérisée en ce que** l'aire de la section transversale de l'orifice d'entrée (42, 52) est plus petite que l'aire de la section transversale de la conduite d'arrivée (30).

14. Procédé de mélange d'au moins deux fluides au moyen d'une buse destinée à des buts médicaux selon la revendication 13, dans lequel les fluides pénètrent avec des débits volumétriques différents dans la chambre de mélange (11), dans lequel le rapport des aires de section transversale des orifices d'entrée (42, 52) correspond au rapport des débits volumétriques, de telle manière que les fluides pénètrent dans la chambre de mélange (11) avec une vitesse d'entrée moyenne essentiellement égale.

15. Dispositif médical, en particulier instrument chirurgical, avec une buse selon l'une quelconque des revendications précédentes.
